# EUROPÄISCHE PATENTSCHRIFT

(11) **EP 3 303 439 B1**
(45) Veröffentlichungstag und Bekanntmachung des Hinweises auf die Patenterteilung: **01.05.2019**
(21) Anmeldenummer: 16732461.5
(22) Anmeldetag: 03.06.2016
(51) Int. Cl.: C08G 18/71, C08G 18/76, C08G 18/28, C08G 18/38, C08G 18/32, C09D 133/00, C07C 271/26, C07C 271/28, C07C 269/02, C07C 237/04, B01F 17/00, C09D 5/00

(54) **AMIDOAMINGRUPPENHALTIGE REAKTIONSPRODUKTE**
REACTION PRODUCTS CONTAINING AMIDO AMINE GROUP
PRODUITS DE RÉACTION CONTENANT LES GROUPES AMIDOAMINE

(30) Priorität: 03.06.2015 EP 15170609
(43) Veröffentlichungstag der Anmeldung: 11.04.2018
(73) Patentinhaber: BYK-Chemie GmbH, 46483 Wesel (DE)
(72) Erfinder: OKKEL, Andreas, 46483 Wesel (DE); OMEIS, Jürgen, 46286 Dorsten-Lembeck (DE); BÖMER, Jörg, 46487 Wesel (DE); ROCH, Monika, 46539 Dinslaken (DE); STELZ, Sabine, 46047 Oberhausen (DE); GÖBELT, Bernd, deceased (DE)
(74) Vertreter: Altana IP Department
(86) Internationale Anmeldenummer: PCT/EP2016/062719
(87) Internationale Veröffentlichungsnummer: WO 2016/193474

(56) Entgegenhaltungen:
- EP-A2- 1 038 896
- WO-A1-2012/101180
- DE-T2- 69 710 935
- US-A1- 2005 043 816
- Isocyanate: "Technical Information ISONATE(TM)143L Typical Properties Nominal Value Unit Test Method", , 3. August 2011 (2011-08-03), XP055296690, Gefunden im Internet: URL:http://catalog.ides.com/docselect.aspx ?I=36808&E=101503&DOC=DOWTDS&DS=123&DK=STD &DC=en [gefunden am 2016-08-22]

## Beschreibung

Die vorliegende Erfindung betrifft amidoamingruppenhaltige Reaktionsprodukte, ein Verfahren zu deren Herstellung, deren Verwendung als Netzmittel, Dispergiermittel und/oder Dispersionsstabilisator und Zusammensetzungen, welche diese enthalten.

Netzmittel, welche gelöst oder dispergiert in einer Flüssigkeit vorliegen, setzen die Oberflächenspannung bzw. die Grenzflächenspannung herab und vergrößern so das Netzvermögen der Flüssigkeit. Auf diese Weise ermöglichen Netzmittel in vielen Fällen erst überhaupt eine Oberflächenbenetzung.

Dispergiermittel eignen sich im Allgemeinen zur Stabilisierung von Feststoffteilchen in Bindemitteln, Lacken, Pigmentpasten, Kunststoffen und Kunststoffmischungen, Kleb- und Dichtstoffen, zur Reduktion der Viskosität entsprechender Systeme sowie zur Verbesserung der Fließeigenschaften. Dispersionsstabilisatoren werden zur Stabilisierung bereits erzeugter Dispersionen eingesetzt.

Um Feststoffe in flüssige Medien einbringen zu können, sind hohe mechanische Kräfte erforderlich. Es ist üblich, Dispergiermittel einzusetzen, um die Dispergierkräfte zu erniedrigen und um den zur Deflockulierung der Feststoffteilchen notwendigen Gesamtenergieeintrag in das System und damit auch die Dispergierzeit so gering wie möglich zu halten. Im Falle derartiger Dispergiermittel handelt es sich um oberflächenaktive Stoffe, anionischer, kationischer und/ oder neutraler Struktur. Diese Stoffe werden in kleiner Menge entweder direkt auf den Feststoff aufgebracht oder dem Dispergiermedium zugesetzt. Wesentlich ist weiterhin, dass auch nach vollständiger Deflockulierung der Feststoffagglomerate in Primärteilchen (nach dem Dispergierprozess) Reagglomerationen auftreten kann, wodurch der Dispergieraufwand teilweise oder vollständig zunichte gemacht wird. Als Folge von unzulänglicher Dispergierung bzw. von Reagglomeration kommt es typischerweise zu unerwünschten Effekten, wie einem Viskositätsanstieg in flüssigen Systemen, einer Farbtondrift und einem Glanzverlust in Lacken und Beschichtungen sowie einer Verringerung der mechanischen Festigkeit und der Materialhomogenität in Kunststoffen.

Als Netz- und Dispergiermittel kommen in der Praxis verschiedenartige Verbindungstypen in Frage. Dies ist insbesondere darin begründet, dass eine hohe Zahl verschiedener Systeme existiert, welche insbesondere auf verschiedenartigen Bindemitteln kombiniert mit unterschiedlichen zu dispergierenden Partikeln, wie Pigmenten, Füllstoffen sowie Fasern, basiert.

Die WO-A-2012/175159 beschreibt beispielswiese die Herstellung von speziellen Additivkompositionen, welche als qualitativ hochwertige Netz- und Dispergiermittel anzusehen sind. Allerdings ist in diesen Additivkompositionen für viele Dispergieraufgaben, insbesondere aufgrund begrenzter Universalität gegenüber den zu dispergierenden Feststoffen, nicht die optimale und vollständige Lösung zu sehen.

Der vorliegenden Erfindung lag somit die Aufgabe zugrunde, qualitativ hochwertige sowie möglichst universell einsetzbare Produkte bereitzustellen, die als Netz- und/oder Dispergiermittel und/oder Dispersionsstabilisatoren geeignet sind.

Die Aufgabe konnte gelöst werden durch Bereitstellung von amidoamingruppenhaltigen Reaktionsprodukten, die eine oder mehrere Spezies der allgemeinen Formel (I) enthalten:

(R¹-X)ₚ-Z¹-(XH)_{y} (I),

wobei gilt p + y = w und
w für eine ganze Zahl von 1 bis 10 steht,
p für eine ganze Zahl von 1 bis 10 steht,
y für eine ganze Zahl von 0 bis 9 steht, und
X unabhängig voneinander für O, NH und/oder NZ² steht und
XH unabhängig voneinander für eine Hydroxylgruppe OH, eine primäre Aminogruppe NH₂ und/oder eine sekundäre Aminogruppe NHZ² steht,
wobei
Z² unabhängig voneinander für einen verzweigten oder unverzweigten, gesättigten oder ungesättigten organischen Rest G(U)ₐ steht, wobei U unabhängig voneinander für eine Hydroxylgruppe, eine primäre Aminogruppe oder eine sekundäre Aminogruppe steht und
a für eine ganze Zahl von 0 bis 9 steht,
wobei gilt p + y + a ≤ 10, und
G für einen verzweigten oder unverzweigten, gesättigten oder ungesättigten organischen Rest steht,
die p Reste R¹ unabhängig voneinander für einen Rest der allgemeinen Formel (II)

   Y-O-CO-NH-R²-NH-CO (II)

   stehen,
   worin die p Reste Y unabhängig voneinander für einen verzweigten oder unverzweigten, gesättigten oder ungesättigten organischen Rest mit 1 bis 1000 Kohlenstoffatomen stehen, der keine Hydroxylgruppen, keine primären Aminogruppen und keine sekundären Aminogruppen enthält,
   die p Reste R² unabhängig voneinander für einen verzweigten oder unverzweigten, gesättigten oder ungesättigten organischen Rest mit 6 bis 20 Kohlenstoffatomen steht, und
   Z¹ für einen mindestens zwei Kohlenstoffatome enthaltenden verzweigten oder unverzweigten, gesättigten oder ungesättigten organischen Rest steht, der mindestens eine Amidgruppe und mindestens eine tertiäre Aminogruppe aufweist.

Vorzugsweise enthält das "amidoamingruppenhaltige Reaktionsprodukt" eine oder mehrere Spezies der allgemeinen Formel (I) als Hauptreaktionsprodukt(e), das heißt die Menge der unter die allgemeine Formel (I) fallenden Spezies des amidoamingruppenhaltigen Reaktionsprodukts beträgt mindestens 40 Gew.-%, bevorzugter mindestens 60 Gew.-% und besonders bevorzugt mindestens 90 Gew.-%, bezogen auf das Gesamtgewicht des amidoamingruppenhaltigen Reaktionsprodukts. Ganz besonders bevorzugt besteht das amidoamingruppenhaltige Reaktionsprodukt im Wesentlichen aus Spezies der allgemeinen Formel (I). Aufgrund der nur kommerziellen Reinheitsgrade der Reaktanten und/oder möglicherweise auftretender Nebenreaktionen bei der Herstellung der amidoamingruppenhaltigen Reaktionsprodukte, ist hierin unter "im Wesentlichen bestehend aus" Spezies der allgemeinen Formel (I) ein Gehalt an Spezies der Formel (I) am Gesamtgewicht des amidoamingruppenhaltigen Reaktionsprodukts von mindestens 95 Gew.-% zu verstehen.

Der Begriff "Spezies" umfasst dabei sowohl Verbindungen mit konkretem Molekulargewicht als auch solche Spezies, die polymere Reste enthalten und somit ein gewichtsmittleres und ein zahlenmittleres Molekulargewicht aufweisen.

Bei den "amidoamingruppenhaltigen Reaktionsprodukten" kann es sich im einfachsten Fall um molekular einheitliche Produkte handeln, wenn ausschließlich molekular einheitliche, chemisch im Wesentlichen reine Verbindungen Y-O-CO-NH-R²-NCO und (HX)ₚ-Z¹-(HX)_{y} zu deren Herstellung eingesetzt werden, in welchen folglich Y nicht polymer ist, alle Y identisch sind, alle R² identisch sind, alle X identisch sind und alle Z¹ identisch sind.

Im einfachsten Fall kann jedoch schon eine molekulare Uneinheitlichkeit der "amidoamingruppenhaltigen Reaktionsprodukte" erhalten werden für den Fall, dass p + y > 1 und dass die Summe aus p und y größer der Anzahl der Spezies Y-O-CO-NH-R²-NCO ist, auch wenn Y nicht polymer ist, alle Y identisch sind, alle R² identisch sind, alle X identisch sind und alle Z¹ identisch sind. In diesem Fall werden molekulare Verteilungen erhalten, indem die "amidoamingruppenhaltigen Reaktionsprodukte" unterschiedliche Spezies der allgemeinen Formel (I) mit verschiedenen Werten für p und y Werten aufweisen.

Bei den "amidoamingruppenhaltigen Reaktionsprodukten" kann es sich jedoch auch um molekular uneinheitliche Produkte handeln. Dies ist beispielsweise dann der Fall wenn der im Rest R¹ enthaltende Rest Y polymerer Natur ist und somit *per se* eine molekulare Uneinheitlichkeit in die Produkte einführt.

Bei den "amidoamingruppenhaltigen Reaktionsprodukten" kann es sich schließlich auch um Mischungen handeln, wenn die Reaktanten beispielsweise unterschiedliche Reste R¹ und/oder Z¹ enthalten. Die Mischungen können Mischungen chemisch einheitlicher amidoamingruppenhaltiger Reaktionsprodukte sein, Mischungen von amidoamingruppenhaltigen Reaktionsprodukten, die polymere Reste enthalten oder Mischungen eines oder mehrerer chemisch einheitlicher amidoamingruppenhaltiger Reaktionsprodukte mit einem oder mehreren amidoamingruppenhaltigen Reaktionsprodukten, die polymere Reste enthalten.

Im Sinne dieser Erfindung sind die Begriffe Hydroxylgruppe sowie primäre und sekundäre Aminogruppe im Sinne ihrer allgemeinen Bedeutung zu verstehen. Dies bedeutet insbesondere, dass sie als eigenständige funktionelle Gruppe vorliegen und nicht Teil einer übergeordneten weiteren funktionellen Gruppe sind. So ist im Sinne dieser Erfindung die OH-Funktionalität einer Carbonsäuregruppe keine Hydroxylfunktionalität.

Die Erfindung betrifft auch ein Verfahren zur Herstellung der erfindungsgemäßen amidoamingruppenhaltigen Reaktionsprodukte beziehungsweise der darin enthaltenen Spezies der allgemeinen Formel (I).

Beim erfindungsgemäßen Verfahren zur Herstellung der erfindungsgemäßen amidoamingruppenhaltigen Reaktionsprodukte wird
i) mindestens ein Diisocyanat R²(NCO)₂ mit mindestens einem Monoalkohol Y-OH unter Ausbildung eines Urethans der allgemeinen Formel (III)

   Y-O-CO-NH-R²-NCO (III)

   umgesetzt, wobei
   R² und Y wie zuvor definiert sind, und
ii) p Urethane der allgemeinen Formel (III) werden mit einer oder mehreren die Gruppe Z¹-(XH)_{y} einbringenden Komponenten zu einem amidoamingruppenhaltigen Reaktionsprodukt, enthaltend ein oder mehrere Spezies gemäß der allgemeinen Formel (I)

   (R¹-X)ₚ-Z¹-(XH)_{y} (I),

   umgesetzt, wobei p, y, R¹, X und Z¹ wie zuvor definiert sind.

### Diisocyanat R²(NCO)₂

Im erfindungsgemäßen Verfahren werden zur Herstellung der erfindungsgemäßen amidoamingruppenhaltigen Reaktionsprodukte Diisocyanate R²(NCO)₂ eingesetzt. Entsprechende Diisocyanate sind aus dem Stand der Technik bekannt. Der zum Diisocyanat R²(NCO)₂ korrespondierende Rest R² ist ein verzweigter oder unverzweigter, gesättigter oder ungesättigter organischer Rest mit 6 bis 20 Kohlenstoffatomen. Der organische Rest R² kann zusätzlich Uretdion- und/oder Urethangruppen enthalten, die allerdings unter den gegebenen Bedingungen nicht zur Reaktion kommen. Dies ist insbesondere der Fall, wenn die Reaktionsmischungen keine primären und/oder sekundären Aminogruppen enthalten. Es ist daher bevorzugt, dass für den Fall, dass R² Uretdion- und/oder Urethangruppen enthält, die Gruppe X durch Sauerstoffatome vertreten wird.

Bevorzugt liegt der organische Rest R² in Form eines Kohlenwasserstoffrests vor, besonders bevorzugt als Arylengruppe, als Alkylarylengruppe und/oder als azyklische, zyklische, verzweigte oder unverzweigte Alkylengruppe.

Derartige Diisocyanate sind zum Beispiel 1,4-Diisocyanatobutan, Hexamethylendiisocyanat (HDI), 2-Methyl-1,5-diisocyanatopentan, 1,5-Diisocyanato-2,2-dimethylpentan, 2,2,4- oder 2,4,4-Trimethyl-1,6-diisocyanatohexan, 1,10-Diisocyanatodecan, 1,3- und 1,4-Diisocyanatocyclohexan, 1,3- und 1,4-Bis-(isocyanatomethyl)-cyclohexan, 1-Isocyanato-3,3,5-trimethyl-5-isocyanatomethylcyclohexan (Isophorondiisocyanat, IPDI), 4,4'-Diisocyanatodicyclohexylmethan (H12MDI), Toluylendiisocyanat (TDI), 1-Isocyanato-1-methyl-4(3)isocyanatomethylcyclohexan, Diphenylmethandiisocyanat (MDI), Bis-(isocyanatomethyl)-norbornan und 1,3- und 1,4-Bis-(2-isocyanato-prop-2-yl)-benzol (TMXDI) oder Mischungen solcher Diisocyanate. Die genannten monomeren Diisocyanate können als solche oder in Form ihrer Uretdion- und/oder Urethan-Gruppen aufweisenden oligomeren oder polymeren Diisocyanatfunktionellen Derivate eingesetzt werden.

Beispiele für als Handelsprodukte erhältliche Diisocyanate sind Desmodur T100 (100%iges 2,4-TDI, Bayer AG), Desmodur T80 (80% 2,4-TDI, 20% 2,6-TDI, Bayer AG), Desmodur T65 (65% 2,4-TDI, 35% 2,6-TDI, Bayer AG), Desmodur N3400 ( Aliphatisches HDI-Uretdion, Bayer AG), Thanecure T9 (Aromatisches TDI-Uretdion, TSE Industries), Crelan VP LS 2147 und Crelan VP LS 2347 (Aliphatische IDPI-Uretdione, Bayer AG). Zur Herstellung der erfindungsgemäßen amidoamingruppenhaltigen Reaktionsprodukte können ein oder mehrere monomere, oligomere oder polymere Diisocyanate verwendet werden.

Zur Erhöhung der Selektivität bezüglich der Herstellung des in Schritt i) erzeugten unter die allgemeine Formel (III) fallenden Urethans, wird im erfindungsgemäßen Verfahren bevorzugt ein Diisocyanat R²(NCO)₂ eingesetzt, welches zwei unterschiedlich reaktive Isocyanatgruppen aufweist. Besonders bevorzugt wird das Diisocyanat R²(NCO)₂ mit zwei unterschiedlich reaktiven Isocyanatgruppen ausgewählt aus der Gruppe bestehend aus Toluol-2,4-diisocyanat und Isophorondiisocyanat.

Bei der Herstellung des Urethans der allgemeinen Formel (III) in Schritt i) wird das Diisocyanat R²(NCO)₂ mindestens im äquimolaren Verhältnis zum Monoalkohol Y-OH eingesetzt. Vorzugsweise erfolgt der Einsatz des Diisocyanats R²(NCO)₂ in Schritt i) im molaren Überschuss, wodurch eine höhere Selektivität dahingehend erreicht wird, dass vorzugsweise nur genau eine NCO-Gruppe des Diisocyanats in Schritt i) umgesetzt wird.

Je größer der molare Überschuss des Diisocyanats, desto höher ist normalerweise die Selektivität bezüglich der Herstellung des in Schritt i) erzeugten unter die allgemeine Formel (III) fallenden Urethans. Das aufgrund eines Einsatzes im Überschuss verbleibende nicht umgesetzte Diisocyanat wird vorzugsweise wenigstens teilweise (jedoch möglichst vollständig) aus dem Reaktionsgemisch, bevorzugt durch Destillation, entfernt, um letztendlich den Anteil daraus entstehender Nebenprodukte möglichst gering zu halten. Vorzugsweise werden wenigstens 75 Mol-%, bevorzugt wenigstens 90 Mol-%, ganz besonders bevorzugt der gesamte Anteil der nicht umgesetzten Teilmenge des Diisocyanats R²(NCO)₂ aus dem Reaktionsgemisch entfernt. Das Diisocyanat verschlechtert die Qualität des Verfahrensprodukts und ist als umweltschädlich anzusehen.

Bevorzugt wird in Schritt i) das Diisocyanat R²(NCO)₂ zum Monoalkohol Y-OH in einem molaren Verhältnis von mindestens 1,1:1,0, besonders bevorzugt von mindestens 2,0:1,0 und ganz besonders bevorzugt von mindestens 2,5:1,0 eingesetzt.

Es ist ganz besonders bevorzugt, dass in Schritt i) sowohl ein oder mehrere Diisocyanate mit unterschiedlich reaktiven Isocyanatgruppen verwendet werden als auch ein molarer Überschuss der Isocyanatkomponente in Bezug auf den Monoalkohol Y-OH eingesetzt wird.

Es ist hervorzuheben, dass die mit dem erfindungsgemäßen Verfahren herstellbaren erfindungsgemäßen amidoamingruppenhaltigen Reaktionsprodukte beziehungsweise die darin enthaltenen Spezies der allgemeinen Formel (I) eine gute Dispergierwirkung bezüglich eines breiten Spektrums von zu dispergierenden Feststoffen zeigen. Dies äußert sich u. a. darin, dass Feststoffe mit sauren, neutralen und basischen Oberflächen jeweils effektiv dispergiert werden können.

Die mit dem erfindungsgemäßen Verfahren herstellbaren amidoamingruppenhaltigen Reaktionsprodukte beziehungsweise die darin enthaltenen Spezies der allgemeinen Formel (I) sind von besonders hoher Qualität und universell als Netz- und Dispergiermittel einsetzbar. Konkret kann gesagt werden, dass die erfindungsgemäß herstellbaren amidoamingruppenhaltigen Reaktionsprodukte beziehungsweise die darin enthaltenen Spezies der allgemeinen Formel (I) sowohl in polaren als auch in unpolaren Bindemittelsystemen erfolgreich eingesetzt werden können und dabei als Netz- und Dispergiermittel bzw. als Dispersionsstabilisatoren eine ausgezeichnete Verträglichkeit zeigen. Dies gewährleistet den erfolgreichen Einsatz in Kombination mit den unterschiedlichsten Bindemitteln und Überzugsmaterialien. Weiterhin ermöglichen die mit dem erfindungsgemäßen Verfahren herstellbaren amidoamingruppenhaltigen Reaktionsprodukte beziehungsweise die darin enthaltenen Spezies der allgemeinen Formel (I) eine flockulationsfreie Mischbarkeit von Pasten, insbesondere Pigmentpasten, bzw. der mit diesen Pasten hergestellten Bindemitteln. Darüber hinaus eignen sich die erfindungsgemäßen amidoamingruppenhaltigen Reaktionsprodukte beziehungsweise die darin enthaltenen Spezies der allgemeinen Formel (I) als Dispersionsstabilisatoren, insbesondere auch als Emulsionsstabilisatoren. Durch den Einsatz der mit dem erfindungsgemäßen Verfahren herstellbaren amidoamingruppenhaltigen Reaktionsprodukte beziehungsweise die darin enthaltenen Spezies der allgemeinen Formel (I) wird die Viskosität des eingetragenen Mahlgutes während der Dispergierung deutlich reduziert und ermöglicht auf diese Weise die Herstellung von Formulierungen, welche einen hohen Feststoffanteil aufweisen. Auf diese Weise kann zur besseren Umweltverträglichkeit der Anteil an (flüchtigen) Lösemitteln reduziert werden. Resümierend kann gesagt werden, dass die mit dem erfindungsgemäßen Verfahren herstellbaren amidoamingruppenhaltigen Reaktionsprodukte beziehungsweise die darin enthaltenen Spezies der allgemeinen Formel (I) bei guter Stabilisierung von Pigmenten oder Füllstoffen die Mahlgutviskosität entsprechender Lacke, Pasten oder Kunststoffformulierungen soweit senken, dass eine Verarbeitung bei hohem Füllgrad möglich ist, ohne dass die Beständigkeit der ausgehärteten Lacke negativ beeinflusst werden. Schließlich ist zu erwähnen, dass das erfindungsgemäße Verfahren verhältnismäßig einfach und wirtschaftlich durchführbar ist, wobei die eingesetzten Ausgangsstoffe allgemein gut verfügbar sind.

### Monohydroxyfunktioneller Alkohol Y-OH

Der im erfindungsgemäßen Verfahren eingesetzte Alkohol Y-OH kann zusätzliche Heteroatome, wie O, S, Si und/oder N, aufweisen, bzw. Ether-, Urethan-, Carbonat-, Amid-, Harnstoff- und/oder Estergruppen enthalten. Die gegenüber Isocyanatgruppen äußerst reaktiven Hydroxylgruppen, primären Aminogruppen und sekundären Aminogruppen dürfen allerdings nicht in Y enthalten sein. Gegebenenfalls ist in den Gruppen Y Wasserstoff gegen Halogen (beispielsweise Fluor und/oder Chlor) substituiert. Der Rest Y kann weitere Gruppen, wie C=C-Doppelbindungen, tragen, die sich bei der Bildung des Additionsproduktes inert verhalten. Die gegebenenfalls vorhandenen Ester, Ether, Urethan, Carbonat und/oder Siloxan-Gruppen können in Blockstruktur vorliegen (beispielsweise Poly(ethylenoxidblock-propylenoxidblock-epsilon-caprolacton), einen Gradienten bilden oder auch statistisch angeordnet sein.

### Y als Etherrest bzw. Polyetherrest:

Als Y-OH können auch Monohydroxypolyether eingesetzt werden. Diese können zum Beispiel durch Alkoxylierung einer monohydroxyfunktionellen Startkomponente T-OH erhalten werden. Prinzipiell kommen alle beschriebenen Komponente Y-OH als Startkomponente T-OH in Frage. Zur Darstellung können beispielsweise Alkanole, Cycloalkanole oder Phenole mit Alkylenoxiden wie Ethylenoxid, Propylenoxid, Butylenoxid, Styroloxid, aliphatischen oder aromatischen Glycidethern wie Isopropylglycidether, Butylglycidether, Allyglycidether, 2-Ethylhexylglycidether, Kresylglycidether und Phenylglycidether umgesetzt werden. Es können auch Mischungen dieser Rohstoffe eingesetzt werden. Im Falle von gemischten Polyethern können diese statistisch, in Gradientenform oder in Blöcken angeordnet sein.

Diese Polyether haben häufig ein zahlenmittleres Molekulargewicht (Mₙ) im Bereich von etwa 100 bis 25000, besonders häufig von 150 bis 15000 und besonders typisch von 200 bis 10000 g/mol.

Bevorzugt sind Polyether auf Basis von Ethylenoxid, Propylenoxid und deren Mischungen.

Beispiele sind hydroxyfunktionelle Vinylverbindungen, wie Hydroxybutylvinylether, monohydroxyfunktionelle Polyoxyalkylenmonoalkohole, wie Allylpolyether (z.B. Polyglykol A 350, Polyglykol A 500, Polyglykol A 1100, Polyglykol A 11-4, Polyglykol A 20-10 oder Polyglykol A 20-20 der Clariant AG oder Pluriol® A 010 R, Pluriol® A 11 RE, Pluriol® A 13 R, Pluriol® A 22 R oder Pluriol® A 23 R der BASF AG), Vinylpolyether (wie Polyglykol V 500, Polyglykol V 1100 oder Polyglykol V 5500 der Clariant AG), Methanol gestartete Polyoxyethylenmonoalkohole (wie Pluriol® A 350 E, Pluriol® A 500 E, Pluriol® A 750 E, Pluriol® A 1020 E, Pluriol® A 2000 E oder Pluriol® A 5010 E der BASF AG), Alkanol gestartete Polyoxypropylenmonoalkohole (wie z.B. Polyglykol B01 / 20, Polyglykol B01 / 40, Polyglykol B01 / 80, Polyglykol B01 / 120 oder Polyglykol B01 / 240 der Clariant AG oder Pluriol® A 1350 P oder Pluriol® A 2000 P der BASF AG) und mit verschiedenen Fettalkoholen gestarteten Polyalkoxylate mit variablem Alkoxylierungsgrad (unter den Handelsnamen Lutensol® A, Lutensol® AT, Lutensol® AO, Lutensol® TO, Lutensol® XP, Lutensol® XL, Lutensol® AP und Lutensol® ON der BASF SE bekannt). Bevorzugt werden Polyoxyalkylenmonoalkohole verwendet, die Ethylenoxid- und/oder Propylenoxid- und/oder Butylenoxidgruppen enthalten und gegebenenfalls mit Styroloxid, Phenylgylcidylether oder Kresylglycidylether modifiziert sind. Besonders bevorzugt ist die Verwendung von Polyoxyalkylenmonoalkoholen (wie Polyglykol B 11/50, Polyglykol B 11/70, Polyglykol B 11/100, Polyglykol B 11/150, Polyglykol B 11/300 oder Polyglykol B 11/700 von der Clariant AG, Pluriol® A 1000 PE, Pluriol® A 1320 PE, oder Pluriol® A 2000 PE der BASF AG oder Terralox WA 110 von DOW Chemicals), bei denen es sich um Butanol gestartete Polyoxyalkylene aus Ethylen- und Propylenoxid mit einer terminalen OH-Gruppe handelt.

In der Regel enthält Y 1 bis 450 Ethersauerstoffatome, welche bevorzugt in Ethersauerstoffatome aufweisenden Gruppen enthalten sind, die sich ableiten von Polytetrahydrofuran, Polyoxetanen und/oder Polyoxiranen.

Bevorzugt enthält Y 3 bis 400 Ethersauerstoffatome, wobei mindestens 50, bevorzugt mindestens 80 Mol-% der Ethersauerstoffatome in Ethylenoxid- und/oder Polypropylenoxid-Struktureinheiten vorliegen.

### Y als Kohlenwasserstoffrest:

Die Kohlenwasserstoffreste liegen bevorzugt als Arylrest, als verzweigter oder unverzweigter Aralkylrest und/oder als acyclischer, cyclischer verzweigter oder unverzweigter Alkylrest vor. Es können auch Gemische solcher Verbindungen, d.h. mindestens zwei verschiedene Verbindungen Y-OH eingesetzt werden. Die aliphatischen oder araliphatischen Verbindungen Y-OH können geradkettig oder verzweigt, gesättigt oder ungesättigt vorliegen. Gesättigte Spezies sind bevorzugt.

Beispiele für Y-OH mit Kohlenwasserstoffresten sind Methanol, Ethanol, Butanol, Ethylhexanol, Decanol, Isotridecylalkohol, Laurylalkohol, Stearylalkohol, Isobornylalkohol, Benzylalkohol, Propargylalkohol, Oleylalkohol, Linoleylalkohol, Oxoalkohole, Neopentylalkohol, Cyclohexanol, Fettalkohole, Alkylphenole, Alkylnaphthole, Phenylethanol.

Weiterhin können Y-OH Polyolefinmonoole, wie nichthydrierte, teilhydrierte und/ oder vollständig hydrierte Polybutadiene, nichthydrierte, teilhydrierte und/ oder vollständig hydrierte Polyisoprene, Polyisobutylene, Polypropylene oder Ethylen/Butylen-Copolymere sein. Diese Verbindungen sind bekannt. So wird beispielsweise der Zugang zu hydroxyfunktionellem Polyisobutylenen in US 6875897 beschrieben.

### Y als Esterrest bzw. Polyesterrest:

Als Y-OH können auch Monohydroxymonoester und Monohydroxypolyester eingesetzt werden.

Hydroxyfunktionelle Acrylate oder Methacrylate, wie Hydroxyethylacrylat, Hydroxyethylmethacrylat, Hydroxybutyl(meth)acrylat sind Beispiele für geeignete Monohydroxymonoester.

Polyester können z.B. durch Umsetzung von Dicarbonsäuren sowie deren veresterbaren Derivaten, wie Anhydriden, Säurechloriden oder Dialkylestern (wie Dimethylestern oder Diethylestern) durch Umsetzung mit Diolen und monofunktionellen Startkomponenten, hergestellt werden. Die Bildung von Dihydroxypolyestern kann bei Bedarf durch Einsatz entsprechend stöchiometrischer Mengen an Monohydroxyverbindungen zurückgedrängt werden. Die Veresterung kann in Substanz oder auch durch azeotrope Veresterung in Anwesenheit eines Schleppmittels durchgeführt werden. Beispiele für Dicarbonsäuren sind Bernsteinsäure, Maleinsäure, Fumarsäure, Glutarsäure, Adipinsäure, Sebacinsäure, Pimelinsäure, Phthalsäure oder dimerisierte Fettsäuren und deren Isomere sowie Hydrierungsprodukte. Beispiele für entsprechende Diole sind: Ethylenglykol, 1,2-Propandiol, 1,3-Propandiol, 1,4-Butandiol, 1,6-Hexandiol, Neopentylglykol, cis-1,2-Cyclohexandimethanol, trans-1,2-Cyclohexandimethanol, sowie Polyglykole auf Basis von Ethylenglykol und/ oder Propylenglykol.

Bevorzugte Polyester für den Einsatz als Y-OH sind solche, die durch Polykondensation einer oder mehrerer, gegebenenfalls alkylsubstituierter, Hydroxycarbonsäuren und/oder Ringöffnungspolymerisation der korrespondierenden Lactone, wie Propiolacton, Valerolacton, Butyrolacton, Caprolacton und/ oder substituierten Lactonen mittels einer Monohydroxy-Startkomponente (wie in US-A-4 647 647 beschrieben) erhalten werden können. Bevorzugt besitzen diese ein zahlenmittleres Molekulargewicht Mₙ von 150 bis 5000 g/mol. Als Startkomponente T-OH sind im Prinzip alle anderen als Y-OH aufgeführten Verbindungen einsetzbar. Es können auch jeweils Gemische der vorgenannten Verbindungen eingesetzt werden. Die Lactonpolymerisation wird nach bekannten Verfahren, initiiert durch beispielsweise Titanate, p-Toluolsulfonsäure oder Dibutylzinndilaurat, bei Temperaturen von etwa 70° C bis 180°C durchgeführt. Besonders bevorzugt sind Polyester auf ε-Caprolacton-Basis, gegebenenfalls in Kombination mit δ-Valerolacton.

### Y als Urethanrest bzw. Polyurethanrest:

Als Y-OH können auch Polyurethane, Polyether- Polyurethane, Polyester-Polyurethane und/oder Polyether-Polyester-Polyurethane eingesetzt werden, die durch Additionsreaktion von Diisocyanaten, mit Dihydroxyverbindungen in Anwesenheit von monofunktionellen Startkomponenten erhalten werden können. Als Hydroxyverbindungen zum Aufbau der urethangruppenhaltigen Verbindungen Y-OH werden bevorzugt Diole mit 2 bis 12 Kohlenstoffatomen, Polyoxyalkylenglykole und dihydroxyfunktionelle Polyester eingesetzt.

### Y als Polycarbonatrest:

Der Rest Y kann auch Carbonatgruppen enthalten, wie diese durch bekannte Umsetzungen mit offenkettigen und/oder cyclischen Carbonaten erhalten werden. Geeignet sind zum Beispiel mit Carbonaten modifizierte lineare Polyester oder Polycarbonatdiole, wie diese in der Polyurethanherstellung verwendet werden. Beispiele sind beschrieben in der US-4 101 529. Geeignete Carbonate sind z.B. aliphatische, cycloaliphatische, araliphatische und/oder aromatische Kohlensäurester, wie Dialkylcarbonate, z.B. Dimethylcarbonat, Diethylcarbonat oder Diphenylcarbonat, Catecholcarbonat oder cyclische Alkylencarbonate. Besonders geeignet sind cyclische Alkylencarbonate mit 5- oder 6-gliedrigen Ringen, die gegebenenfalls substituiert sein können. Als Substituenten sind aliphatische, cycloaliphatische und/oder aromatische Gruppen mit bis zu 30 Kohlenstoffatomen bevorzugt. Beispiele für geeignete cyclische Alkylencarbonate sind Ethylencarbonat, Propylencarbonat, Glycerincarbonat, Trimethylencarbonat, 4-Methyltrimethylencarbonat, 5-Methyltrimethylencarbonat, 5,5-Dimethyltrimethylencarbonat, 5,5-Diethyltrimethylencarbonat oder 5-Methyl-5-propyltrimethylencarbonat.

### Y als Polyoxazolinrest:

Als Y-OH können auch monohydroxyfunktionelle Poly-2-alkyl-2-oxazoline oder Poly-2-alkyl-2-oxazine fungieren. Poly-2-alkyl-2-oxazoline oder Poly-2-alkyl-2-oxazine werden durch kationische, ringöffnende Polymerisation von 2-Alkyl-2-oxazolinen oder 2-alkyl-2-oxazinen mit Initiatoren, wie Para-Toluolsulfonsäure, Methyltosylat oder Methyltriflat, erhalten. Die durch den lebenden kationischen Polymerisationsmechanismus resultierenden Oxazolinium- oder Oxaziniumendgruppen können durch alkalische Hydrolyse über Aminoesterendgruppen in die stabileren Hydroxyamide überführt werden. Ein alternativer Weg zur Herstellung von monohydroxyfunktionellen Poly-2-alkyl-2-oxazolinen oder Poly-2-alkyl-2-oxazinen ist die Polymerisation mit 2-(4-hydroxyphenyl)-N-methyl-2-oxazolinium trifluormethansulfonat als initiierender Spezies. (A. Groß, G. Maier, O. Nuyken, Macromol. Chem. Phys. 197, 2811-2826 (1996)). Durch Wahl des Alkylsubstituenten lässt sich die Verträglichkeit steuern, so ist beispielsweise Poly-2-ethyl-2-oxazolin durch seine Wasserlöslichkeit für hochpolare Systeme geeignet, während beispielsweise Poly-2-lauryl-2-oxazolin in unpolaren Systemen verträglich ist. Werden Blockcopolymere aus 2-Ethyl-2-oxazolin und 2-Lauryl-2-oxazolin gebildet, so zeichnen sich die Polymere durch eine besonders breite Verträglichkeit aus. Solche Poly-2-alkyl-2-oxazoline oder Poly-2-alkyl-2-oxazine besitzen meist ein zahlenmittleres Molekulargewicht Mₙ von 300 bis 20000 g/mol, bevorzugt von 500 bis 10000 g/mol. Einsetzbar sind unter anderem verschiedenartige 2-Oxazoline, welche eventuell zusätzliche funktionelle Gruppen aufweisen können. Derartige Spezies sind beispielsweise entsprechende fettsäurebasierte 2-Oxazoline.

### Y als OH-funktionelles Polymer ethylenisch ungesättigter Verbindungen:

Als Y-OH können auch OH-funktionelle Polymere von ethylenisch ungesättigten Monomeren eingesetzt werden. Die OH-Funktionen können über monohydroxyfunktionelle ethylenisch ungesättigte Monomere, über monohydroxyfunktionelle Initiatoren oder monohydroxyfunktionelle Kettenregler in bekannter Weise eingeführt werden, wie beipielsweise bei der radikalischen Polymerisation. Besonders hohe Selektivitäten in Bezug auf die Monohydroxyfunktionalität lassen sich durch kontrollierte, beziehungsweise lebende Polymerisationsverfahren erzielen wie zum Beispiel ATRP, NMP, RAFT oder anionische Polymerisation. Bevorzugt sind monohydroxyfunktionelle Polyacrylsäureester bzw. Polymethacrylsäureester. Solche Verbindungen sind auf diesem Gebiet der Technik bereits zur Herstellung von anderen Dispergiermitteln verwendet worden, wie sie in der US-A-4 032 698 bzw. in der EP 318 999 beschrieben sind. Beispielsweise sind monohydroxyfunktionelle Polyacrylatmakromere wie Actflow UMM 1001 der Firma Soken Chemical & Engineering Co. kommerziell erhältlich. Diese Polyacrylate haben meist ein zahlenmittleres Molekulargewicht Mₙ von 300 bis 20000 g/mol, bevorzugt meist von 500 bis 10000 g/mol. Diese können in Blockstruktur oder auch statistisch angeordnet sein oder einen Gradienten bilden.

Beispiele für OH-funktionelle ethylenisch ungesättigte Monomere sind Hydroxyalkyl(meth)acrylate von geradkettigen, verzweigten oder cycloaliphatischen Diolen mit 2 bis 36 C-Atomen, wie 3-Hydroxypropylmethacrylat, 3,4-Dihydroxybutylmonomethacrylat, 2-Hydroxyethyl(meth)acrylat, 4-Hydroxybutyl(meth)acrylat, 2-Hydroxypropylmethacrylat, 2,5-Dimethyl-1,6-hexandiolmonomethacrylat; Caprolacton- und/oder Valerolacton-modifizierte Hydroxyalkyl(meth)acrylate (wobei die Hydroxy(meth)acrylate bevorzugt von geradkettigen, verzweigten oder cycloaliphatischen Diolen mit 2 bis 8 C-Atomen abgeleitet sind); OH-funktionelles Poly(ethylenglycol)(meth)acrylat und OH-funktionelles Poly(propylenglycol)(meth)acrylat.

Beispiele für weitere ethylenisch ungesättigte Monomere sind Alkyl(meth)acrylate von geradkettigen, verzweigten oder cycloaliphatischen Alkoholen mit 1 bis 22 Kohlenstoffatomen, wie Methyl(meth)acrylat, Ethyl(meth)acrylat, n-Butyl(meth)acrylat, i-Butyl(meth)acrylat, t-Butyl(meth)acrylat, Lauryl(meth)acrylat, 2-Ethylhexyl(meth)acrylat, Stearyl(meth)acrylat, Cyclohexyl(meth)acrylat, lsobornyl(meth)acrylat und t-Butyl(meth)acrylat; Aralkyl(meth)acrylate, wie Benzylmethacrylat und Aryl(meth)acrylate wie Phenylacrylat (wobei die Arylreste jeweils unsubstituiert oder bis zu vierfach substituiert sein können), wie 4- Nitrophenylmethacrylat;
Mono(meth)acrylate von Ethern, Polyethylenglykolen, Polypropylenglycolen oder gemischten Polyethylen/propylenglycolen mit 5 bis 80 Kohlenstoffatomen, wie Tetrahydrofurfurylmethacrylat, Methoxyethoxyethylmethacrylat, 1-Butoxypropylmethacrylat, Cyclohexyloxymethylmethacrylat, Methoxymethoxyethylmethacrylat, Benzyloxymethylmethacrylat, Furfurylmethacrylat, 2- Butoxyethylmethacrylat, 2-Ethoxyethylmethacrylat, Allyloxymethylmethacrylat, 1-Ethoxybutylmethacrylat, 1-Ethoxyethylmethacrylat, Ethoxymethylmethacrylat, Poly(ethylenglycol)methylether(meth)acrylat, Poly(propylenglycol)methylether(meth)acrylat; tertiäre Aminoalkyl(meth)acrylate, wie N, N-Dimethylaminoethyl(meth)acrylat, 2-Trimethylammoniumethylmethacrylatchlorid und N,N-Dimethylaminopropyl(meth)acrylat; (Meth)acrylate von halogenierten Alkoholen, wie Perfluoralkyl(meth)acrylate mit 6 bis 20 C-Atomen; Styrol und substituierte Styrole, wie 4-Methylstyrol, Methacrylnitril und Acrylnitril;
ethylenisch ungesättigte Heterocyclen, wie zum Beispiel 4-Vinylpyridin und 1-[2-(Methacryloyloxy)-ethyl]-2-imidazolidinon;
Vinylester von Carbonsäuren mit 1 bis 20 C-Atomen, wie Vinylacetat;
Maleinimid, N-Phenylmaleinimid und N-substituierte Maleinimide mit geradkettigen, verzweigten oder cycloaliphatischen Alkylgruppen mit 1 bis 22 C-Atomen, wie N-Ethylmaleinimid und N-Octylmaleinimid; (Meth)acrylamid; N-Alkyl- und N,N-Dialkylsubstituierte Acrylamide mit geradkettigen, verzweigten oder cycloaliphatischen Alkylgruppen mit 1 bis 22 C-Atomen, wie N-(t-Butyl)acrylamid und N,N-Dimethylacrylamid;

Bevorzugte nicht OH-funktionelle Monomere sind Alkyl(meth)acrylate, Aryl(meth)acrylate, Aralkyl(meth)acrylate und Styrol.

### Y als Polysiloxanrest:

Als Y-OH können auch monohydroxyfunktionelle Polysiloxane eingesetzt werden. Die Polysiloxane lassen vorzugsweise sich mit der nachstehenden allgemeinen Formel beschreiben: 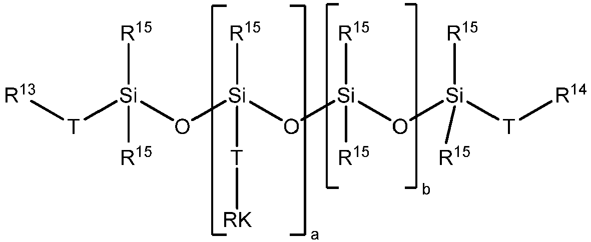 mit
- T: = C₁-C₁₄-Alkylen,
- RK: = unverzweigter Polyetherrest aus Alkylenoxid-Einheiten mit 1-6 Kohlenstoffatomen, und/oder aliphatischer und/oder cycloaliphatischer und/oder aromatischer Polyesterrest mit einem gewichtsmittleren Molekulargewicht zwizwischen 200 und 4000 g/mol,
- R¹³ und R¹⁴: jeweils unabhängig repräsentiert durch
C₁-C₁₄-Alkyl, -Aryl oder -Aralkyl, -O(C₁-C₁₄-Alkyl, -Aryl oder -Aralkyl), -OCO(C₁-C₁₄-Alkyl, -Aryl oder -Aralkyl), -O-CO-O(C₁-C₁₄-Alkyl, -Aryl oder -Aralkyl), -OSO₂(C₁-C₁₄-Alkyl, -Aryl oder -Aralkyl), -H, -Cl, -F, -OH, -R, -RK,
- R¹⁵: = C₁-C₁₄-Alkyl, -Aryl oder -Aralkyl,
- a: = 0 - 20, bevorzugt 1 - 15, besonders bevorzugt 1 - 8,
- b: = 2 - 300, bevorzugt 10 - 200, besonders bevorzugt 15 - 100 und
Die Polysiloxanreste können auch als organomodifizierte Polysiloxanreste vorliegen.

### Herstellung von Urethanen der allgemeinen Formel (III) aus Y-OH und R²(NCO)₂

Die Urethane der allgemeinen Formel (III) werden im Folgenden, insbesondere in den Beispielen, auch als Monoaddukte **M** bezeichnet.

In dem erfindungsgemäßen Verfahren kann die **Isocyanataddition**, je nach Reaktivität der einzelnen Reaktionspartner, in dem für diese Art von Reaktion üblichen Temperaturbereich von Raumtemperatur bis etwa 150 °C, bevorzugt bis 100°C, besonders bevorzugt bis 70°C erfolgen. Zur Beschleunigung und Reduzierung von Nebenreaktionen können die bekannten und üblichen Katalysatoren, wie tertiäre Amine, Triethylamin, Dimethylcyclohexylamin, N-Methylmorpholin, N,N'-Dimethylpiperazin, 2-(Dimethylaminoethoxy)ethanol, Diazabicyclo-(2,2,2)octan und ähnliche sowie insbesondere organische Metallverbindungen, wie Titansäureester, Eisenverbindungen wie Eisen-(III)-acetylacetonat, Zinnverbindungen, beispielsweise Zinndiacetat, Zinndioctoat, Zinndilaurat oder die Dialkylderivate von Zinndialkylsalzen aliphatischer Carbonsäuren wie Dibutylzinndiacetat, Dibutylzinndilaurat oder ähnliche verwendet werden. Diese Katalysatoren werden üblicherweise in Mengen von 0,0001 bis 0,1 Gew.-Teilen pro 100 Gew.-Teile Diisocyanat eingesetzt.

### Amidoamingruppenhaltiges Reaktionsprodukt (R¹-X)ₚ-Z¹-(XH)_{y}

Das erfindungsgemäße amidoamingruppenhaltige Reaktionsprodukt enthält ein oder mehrere Spezies der allgemeinen Formel (I):

(R¹-X)ₚ-Z¹-(XH)_{y} (I),

wobei gilt p + y = w, und
   w für eine ganze Zahl von 1 bis 10 steht,
   p für eine ganze Zahl von 1 bis 10 steht und
   y für eine ganze Zahl von 0 bis 9 steht.
X steht unabhängig voneinander für O, NH und/oder NZ² und die Gruppe XH steht unabhängig voneinander für eine Hydroxylgruppe OH, eine primäre Aminogruppe NH₂ und/oder eine sekundäre NHZ². Besonders bevorzugt steht X für O und die Gruppe XH ist eine Hydroxylgruppe OH.
Z² steht unabhängig voneinander für einen verzweigten oder unverzweigten, gesättigten oder ungesättigten organischen Rest G(U)ₐ, wobei U unabhängig voneinander für eine Hydroxylgruppe, eine primäre Aminogruppe oder eine sekundäre Aminogruppe steht und
a für eine ganze Zahl von 0 bis 9 steht,
wobei gilt p + y + a ≤ 10.

Die Gruppe G steht für einen verzweigten oder unverzweigten, gesättigten oder ungesättigten organischen Rest, wobei dieser bevorzugt 1 bis 30 Kohlenstoffatome, bevorzugter 1 bis 18 und besonders bevorzugt 1 bis 10 Kohlenstoffatome enthält.

Der organische Rest G weist keine gegenüber Isocyanatgruppen reaktive Gruppen HX auf. Der organische Rest G kann jedoch gegebenenfalls auch Heteroatome, insbesondere Sauerstoff und/oder Stickstoffatome, enthalten. Diese liegen bevorzugt in Form von Ethersauerstoffatomen und/oder tertiären Aminogruppen vor.

Über die Begrenzung der Summe von p + y + a auf kleiner gleich 10 wird berücksichtigt, dass der bzw. die mögliche/n organische/n Rest/e Z² auch gegenüber Isocyanatgruppen reaktive Gruppen HX aufweisen kann/können. Es ist jedoch erfindungswesentlich, dass eine Spezies der allgemeinen Formel (I) maximal so viele Gruppen HX enthält, dass die Summe aller y + a HX Gruppen und aller p Reste R¹-X eine maximale Anzahl von 10 nicht überschreitet.

Die in obiger Formel (I) enthaltende Gruppe Z¹ kann im allgemeinen als Kopfgruppe bezeichnet werden. Diese Kopfgruppe Z¹ repräsentiert einen mindestens zwei Kohlenstoffatome enthaltenden verzweigten oder unverzweigten, gesättigten oder ungesättigten organischen Rest, der mindestens eine Amidgruppe und mindestens eine tertiäre Aminogruppe enthält. Bevorzugt enthält die Kopfgruppe Z¹ 2 bis 50 Kohlenstoffatome, bevorzugter 2 bis 30 Kohlenstoffatome und besonders bevorzugt 2 bis 20 Kohlenstoffatome.

### Komponente (HX)ₚ-Z¹-(HX)_{y}

Die zur Formel (I) korrespondierende, urethangruppenfreie, gegenüber Isocyanatgruppen reaktive Spezies liegt gemäß der allgemeinen Formel (IV) vor

(HX)ₚ-Z¹-(HX)_{y} (IV),

wobei p, y, X und Z¹ wie für die allgemeine Formel (I) definiert sind. Spezies der allgemeine Formel (IV) sind im Allgemeinen nicht kommerziell erhältlich. Sie werden vorzugsweise durch Umsetzung einer oder mehrerer Komponenten A mit einer oder mehrerer Komponenten B und/ oder C erhalten.

Die Spezies der allgemeinen Formel (IV) werden im Folgenden, insbesondere in den Beispielen, auch als Amidoamine **AA** bezeichnet.

Die **Komponente A** wird unabhängig voneinander gewählt aus der Gruppe ethylenisch ungesättigter Carbonsäuren, deren Estern und deren Säurehalogeniden, wobei mindestens eine C=C-Doppelbindung und mindestens eine C=O-Doppelbindung konjugiert vorliegen und die C=O-Doppelbindung gewählt wird aus der Gruppe der Carbonsäuren, der Carbonsäureester und Carbonsäurehalogenide.

Vorzugsweise werden als Komponente A ethylenisch ungesättigte Carbonsäureester eingesetzt.

Beispiele für ethylenisch ungesättigte Esterverbindungen, bei denen die C=C-Doppelbindung und die C=O-Doppelbindung der Estergruppe konjugiert sind, sind Alkylacrylate, wie zum Beispiel Methylacrylat, Ethylacrylat, n-Propylacrylat, i-Propylacrylat, n-Butylacrylat, i-Butylacrylat, t-Butylacrylat, 2-Ethylhexylacrylat, Cyclohexylacrylat und Laurylacrylat; Monoacrylate von kurzkettigen Ethern, wie Tetrahydrofurfurylacrylat, Methoxyethoxyethylacrylat, 1-Butoxypropylacrylat, Cyclohexyloxymethylacrylat, Methoxymethoxyethylacrylat, Benzyloxymethylacrylat, Furfurylacrylat, 2-Butoxyethylacrylat, 2-Ethoxyethylacrylat, Allyloxymethylacrylat, 1-Ethoxybutylacrylat, 1-Ethoxyethylacrylat, Ethoxymethylacrylat; Maleinsäurediester, wie Dimethylmaleinat, Diethylmaleinat und Dibutylmaleinat; Fumarsäurediester, wie Dimethylfumarat, Diethylfumarat und Dibutylfumarat; Itakonsäureester, wie Itakonsäuredimethylester und Itakonsäurediethylester.

Bevorzugt wird die Komponente A ausgewählt aus den Acrylsäurestern, besonders bevorzugt aus den kurzkettigen Alkylacrylaten mit einer C₁- bis C₆-Alkylkette. Ganz besonders bevorzugt sind Methylacrylat und Ethylacrylat.

Die **Komponente B** liegt gemäß der allgemeinen Formel (V) vor

(R³)ₓ-HN-(R⁴)_{z} (V),

wobei gilt x + z = 2 und
   x für eine ganze Zahl von 0 bis 2 steht,
   z für eine ganze Zahl von 0 bis 2 steht, und
R³ unabhängig voneinander für H oder einen verzweigten oder unverzweigten, gesättigten oder ungesättigten organischen Rest mit 1 bis 12 Kohlenstoffatomen steht, und
R⁴ unabhängig voneinander für einen verzweigten oder unverzweigten, gesättigten oder ungesättigten organischen Rest mit 2 bis 12 Kohlenstoffatomen und 1 bis 3 tertiären Aminogruppen steht, der gegebenenfalls noch 1 bis 3 primäre und/oder sekundäre Aminogruppen aufweisen kann.

Liegen mehrere Komponenten B vor, so werden diese unabhängig voneinander durch die obige Formel wiedergegeben.

Beispiele für die Komponente B, bei denen x den Wert 2 annimmt, sind Butylamin, Hexylamin, Dibutylamin, Diethylamin, Dipropylamin, Benzylamin, N-Benzylmethylamin und N-Phenylbenzylamin.

Beispiele für die Komponente B, bei denen x den Wert 1 annimmt, sind N,N-Dimethylaminoethylamin, N,N-Dimethylaminopropylamin, N,N-Diethylaminoethylamin, N,N-Diethylaminopropylamin, Tris[2-(methylamino)ethyl]amin, N,N-Dimethyldipropylenetriamin, N,N-Bis(3-aminopropyl)methylamin, Tris(3-aminopropyl)amin, Tris(2-aminoethyl)amin, 2-(2-Methylaminoethyl)pyridin, 2-Aminomethylpyridin, 4-Aminomethylpyridin,1-(3-Aminopropyl)imidazol und N,N,N'-Trimethylendiamin.

Ein Beispiel für die Komponente B, bei dem x den Wert 0 annimmt, ist Bis(3-dimethylaminopropyl)amin.

Die **Komponente C** liegt gemäß der allgemeinen Formel (VI) vor

(R⁵)ₖ-HN-(R⁶)ₙ (VI),

wobei gilt k + n = 2 und
   k für eine ganze Zahl von 0 bis 1 steht,
   n für eine ganze Zahl von 1 bis 2 steht, und
R⁵ für H oder einen verzweigten oder unverzweigten, gesättigten oder ungesättigten organischen Rest mit 1 bis 12 Kohlenstoffatomen steht, und
R⁶ unabhängig voneinander für einen verzweigten oder unverzweigten, gesättigten oder ungesättigten organischen Rest mit 2 bis 12 Kohlenstoffatomen und 1 bis 4 Hydroxylgruppen steht, der gegebenenfalls eine tertiäre Aminogruppe enthält.

Liegen mehrere Komponenten **C** vor, so werden diese unabhängig voneinander durch die obige Formel wiedergegeben.

Beispiele für die Komponente C, bei denen k den Wert 0 annimmt, sind Diethanolamin, Diisopropanolamin, Dipropanolamin, N-(2'-Hydroxyethyl)piperazin, 3-((2-Hydroxyethyl)amino)-1-propanol.

Beispiele für die Komponente C, bei denen k den Wert 1 annimmt, sind Ethanolamin, Propanolamin, 4-Amino-1-butanol, 3-Amino-1-butanol, 1-Aminopropan-2-ol, 5-Amino-1-pentanol, N-(2-Hydroxyethyl)-N-methyl-1,3-propylenediamin, 2-Amino-2-ethyl-1,3-propandiol, N-Methylethanolamin, N-Ethylethanolamin, N-Butylethanolamin, N-(2-Hydroxyethyl)anilin, 1,1,1-Tris(hydroxymethyl)-methanamin, Aminopropyldiethanolamin, Glucosamin und 2-(2-Aminoethoxy)ethanol.

Vorzugsweise erfolgt die Herstellung der Spezies der allgemeinen Formel (IV) in zwei Schritten.

Der erste Schritt ist dabei die Addition einer primären oder sekundären Aminogruppe einer Komponente B und/oder C an die C=C-Doppelbindung einer Komponente A im Rahmen einer Michaeladditionsreaktion. Das entsprechende Produkt ist ein Michaeladditionsprodukt und wird im Folgenden als Intermediat I bezeichnet. Diese Umsetzungen erfolgen vorzugsweise in einem Temperaturbereich von 0 bis 100 °C, bevorzugter von 10 bis 80 °C und besonders bevorzugt von 15 bis 50 °C.

Primäre Aminogruppen sind in der Lage zwei Mal im Rahmen einer Michaeladdition reagieren zu können. Dazu addiert zunächst die primäre Aminogruppe einer Komponente B oder C an die C=C-Doppelbindung einer Komponente A unter Ausbildung einer sekundären Aminogruppe. Diese sekundäre Aminogruppe ist in der Lage im Rahmen einer weiteren Michaeladdition mit einer C=C-Doppelbindung einer Komponente A unter Ausbildung einer tertiären Aminogruppe zu reagieren. Auch das zweifache Michaeladditionsprodukt einer Komponente B oder C mit zwei unabhängig voneinander ausgewählten Komponenten A ist ein Intermediat I wie zuvor beschrieben.

Im zweiten Reaktionsschritt wird mindestens eine der im Intermediat I vorhandenen C=O-Doppelbindungen gewählt aus der Gruppe der Carbonsäuren, der Carbonsäureester und der Carbonsäurehalogenide mit einer primären oder sekundären Aminogruppe einer Komponente B oder C im Rahmen einer Amidierungsreaktion unter Ausbildung einer Spezies der allgemeinen Formel (IV) umgesetzt. Es ist bevorzugt, dass die im Intermediat vorhandene C=O-Doppelbindung als Carbonsäurestergruppe vorliegt.

Diese Umsetzungen erfolgen vorzugsweise in einem Temperaturbereich von 50 bis 180 °C, bevorzugter von 70 bis 160 °C und besonders bevorzugt von 80 bis 150 °C.

Wenn das Intermediat I mehr als eine C=O-Doppelbindungen gewählt aus der Gruppe der Carbonsäuren, der Carbonsäureester und der Carbonsäurehalogenide aufweist, können auch diese in einer Amidierungsreaktion mit einer primären oder sekundären Aminogruppe zu einer Amidgruppe umgesetzt werden.

Die in der Amidierungsreaktion gebildeten Spaltungsprodukte, wie z.B. im Falle der bevorzugten Carbonsäureesterspaltung gebildete Alkohol, werden während oder nach der Reaktion aus dem Reaktionsgemisch entfernt. Des Weiteren wird das gegebenenfalls bei der Reaktion eingesetzte Lösemittel aus dem Reaktionsgemisch entfernt.

Es ist beispielsweise auch möglich, dass zur Herstellung einer Spezies der allgemeinen Formel (IV) Edukte eingesetzt werden, die bereits ein Intermediat I darstellen, so dass diese in einer oder mehreren Amidierungsreaktionen mit einer oder mehreren primären oder sekundären Aminogruppen der Komponenten B und/oder C zu Produkten gemäß der allgemeinen Formel (IV) umgesetzt werden können.

Es ist beispielsweise auch möglich, dass zur Herstellung einer Spezies der allgemeinen Formel (IV) Edukte zum Einsatz kommen, die mindestens eine Amidgruppe und mindestens eine C=C-Doppelbindung aufweisen, die in Konjugation mit der C=O-Doppelbindung der Amidgruppe vorliegt. Spezies der allgemeinen Formel (IV) werden nun durch Michaeladdition einer primären oder sekundären Aminogruppe der Komponente B oder C an die C=C-Doppelbindung hergestellt.

Die Auswahl der Komponenten B gemäß Formel (V) und/oder C gemäß der allgemeinen Formel (VI) zur Herstellung einer Spezies der allgemeinen Formel (IV) ist nicht weiter eingeschränkt und unterliegt lediglich der Vorgabe, dass das resultierende Produkt eine Spezies der allgemeinen Formel (IV) darstellt. Dies bedeutet, dass das Produkt 1 bis 10 gegenüber Isocyanatgruppen reaktive Gruppen enthält sowie mindestens eine tertiäre Aminogruppe und mindestens eine Amidgruppe enthält.

### Verfahrensschritt ii) des erfindungsgemäßen Verfahrens

Schritt ii) des erfindungsgemäßen Verfahrens, kann entweder in einer Stufe ii-a) oder in einer Stufenabfolge ii-b) durchgeführt werden.

Wenn der Verfahrensschritt ii) des erfindungsgemäßen Verfahrens in einer Stufe ii-a) durchgeführt wird, werden p Urethane der allgemeinen Formel (III) mit einer urethangruppenfreien, gegenüber Isocyanatgruppen reaktiven Spezies der allgemeinen Formel (IV) zu einem amidoamingruppenhaltigen Reaktionsprodukt, enthaltend ein oder mehrere Spezies gemäß der allgemeinen Formel (I), umgesetzt.

Die Isocyanataddition kann vorzugsweise, je nach Reaktivität der einzelnen Reaktionspartner, in dem für diese Art von Reaktion üblichen Temperaturbereich von Raumtemperatur bis etwa 150 °C, bevorzugt bis 100 °C, besonders bevorzugt bis 70 °C erfolgen.

In der Stufenabfolge ii-b) werden p Urethane der allgemeinen Formel (III) zunächst mit einem Intermediat I unter Ausbildung eines Zwischenproduktes J umgesetzt. Wie zuvor beschrieben ist das Intermediat I vorzugsweise ein Michaeladditionsprodukt mindestens einer Komponente A mit einer Komponente B oder C. Zur Durchführung der Stufenabfolge ii-b) ist es erfindungswesentlich, dass das Intermediat I mindestens eine gegenüber Isocyanatgruppen reaktive Gruppe HX, mit X wie für die allgemeine Formel (I) definiert, aufweist. Das Zwischenprodukt J wird durch Reaktion einer Isocyanatgruppe mindestens eines Urethans der allgemeinen Formel (III) mit mindestens einer Gruppe HX des Intermediats I gebildet. Das so entstehende Zwischenprodukt J enthält mindestens eine C=O-Doppelbindung ausgewählt aus der Gruppe der Carbonsäuren, der Carbonsäureester und der Carbonsäurehalogenide, wobei Carbonsäureester bevorzugt sind.

Durch eine bzw. mehrere entsprechende Amidierungsreaktionen mit einer oder mehreren Komponenten B und/oder C erfolgt neben der Ausbildung der Kopfgruppe Z¹ gleichzeitig die Ausbildung eines amidoamingruppenhaltigen Reaktionsproduktes enthaltend ein oder mehrere Spezies der allgemeinen Formel (I). Diese Umsetzungen erfolgen vorzugsweise in einem Temperaturbereich von 50 bis 180 °C, bevorzugter von 70 bis 160 °C und besonders bevorzugt von 80 bis 150 °C.

Die in Schritt ii) erfolgte Umsetzung der Isocyanatgruppe(n) des Urethans der allgemeinen Formel (III) sollte möglichst vollständig erfolgen. Optimalerweise sind die hergestellten amidoamingruppenhaltigen Reaktionsprodukte weitgehend frei von Isocyanatgruppen, insbesondere weitgehend frei von dem eingesetzten Diisocyanat des Schritts i).

Die nach dem erfindungsgemäßen Verfahren hergestellten - nicht nachbehandelten bzw. aufgereinigten - amidoamingruppenhaltigen Reaktionsprodukte enthalten typischerweise geringe Mengen Diurethan und Diharnstoff, die aufgrund von Nebenreaktionen entstehen. Mögliche Nebenreaktionen sind die zweifache Reaktion eines Diisocyanats R²(NCO)₂ mit einem Monoalkohol Y-OH unter Ausbildung eines Diurethans der allgemeinen Formel Y-O-CO-NH-R²-NH-CO-O-Y sowie die zweifache Reaktion eines Diisocyanats R²(NCO)₂ mit einer Spezies der allgemeinen Formel (IV) unter Ausbildung einer oder mehrerer Spezies der allgemeinen Formel (HX)_{y+(p-1)}-Z¹-(X-CO-NH-R²-NH-CO-X)-Z¹-(XH)_{y+(p-1)}. Je nach Definition der Gruppe X entstehen in der letzteren Reaktion für X = O Diurethane, für X = NH oder NZ² Diharnstoffe und für den Fall, dass X verschieden durch O und NH/NZ² dargestellt wird Harnstoffurethane. Entsprechend geringe Mengen dieser Nebenprodukte verursachen keinerlei Verschlechterung bezüglich einer Verwendung als Netz- und Dispergiermittel und sind ein deutlicher Hinweis darauf, dass die amidoamingruppenhaltigen Reaktionsprodukte unter Einsatz von Monohydroxyverbindungen mit dem erfindungsgemäßen Verfahren hergestellt worden sind. Des Weiteren können die erfindungsgemäßen amidoamingruppenhaltigen Reaktionsprodukte auch noch geringe Menge des nicht umgesetzten Urethans der Formel (III) enthalten. Durch den Einsatz entsprechender Mengen einer Spezies der allgemeinen Formel (IV) in Schritt ii-a) bzw. des Intermediats I in Schritt ii-b) des erfindungsgemäßen Verfahrens kann der Anteil des Urethans der allgemeinen Formel (III) in der Regel zumindest fast gegen Null reduziert werden, was für die Qualität der erfindungsgemäßen amidoamingruppenhaltigen Reaktionsprodukte meist vorteilhaft ist. Entsprechende Reaktionen nach Schritt ii) des erfindungsgemäßen Verfahrens gelten vorzugsweise als beendet, wenn ein NCO-Gehalt von < 0,1% ermittelt wird. Die erfindungsgemäßen amidoamingruppenhaltigen Reaktionsprodukte sind umweltfreundlich, gut lagerfähig und zeigen - entsprechend wie die erfindungsgemäßen Spezies der allgemeinen Formel (I) als solches - ausgezeichnete Eigenschaften als Netz- und Dispergiermittel.

### Lösemittel

Das erfindungsgemäße Verfahren kann, je nach Viskosität, in Substanz oder in Gegenwart von geeigneten Lösemitteln, Lösemittelgemischen oder anderen geeigneten Trägermedien durchgeführt werden. Geeignet sind alle Lösemittel oder Trägermedien, die unter den gewählten Reaktionsbedingungen nicht reaktiv sind oder deren Reaktivität gegenüber den Reaktionspartnern zu vernachlässigen ist und in denen die Reaktanden und die Reaktionsprodukte zumindest teilweise löslich sind. Hierzu zählen beispielsweise Kohlenwasserstoffe wie Toluol, Xylol, aliphatische und/oder cyloaliphatische Benzinfraktionen, chlorierte Kohlenwasserstoffe wie Chloroform, Trichlorethan, cyclische und acyclische Ether wie Dioxan, Tetrahydrofuran, Polyalkylenglykoldialkylether wie Dipropylenglykoldimethylether, Ester von Mono-, Di- oder Polycarbonsäuren wie Ethylacetat, Butylacetat, Butyrolacton, Dimethyl-2-Methylglutarat, Triacetin, Phthalate oder andere Weichmacher, Di- oder Polycarbonsäureester, als "Dibasic Ester" bezeichnete Dialkylester von C₂-C₄-Dicarbonsäuren, Alkylglykolester wie Ethylglykolacetat, Methoxypropylacetat, Ketone wie Methylisobutylketon, Cyclohexanon, Aceton, Säureamide wie Dimethylformamid, N-Methylpyrrolidon und dergleichen.

Die Lösemittel sollten für die Durchführung der oben beschriebenen Reaktionen zweckmäßiger Weise so ausgewählt werden, dass sich diese unter den Reaktionsbedingungen inert gegenüber dem Reaktanten verhalten.

Zweckmäßig wählt man das oder die Lösemittel bzw. Trägermedien bereits unter Berücksichtigung des geplanten Einsatzgebietes aus. Zum Beispiel werden für den Einsatz in wasserverdünnbaren Lacksystemen oder zur Belegung von Pigmenten in wässriger Suspension nach der Pigmentsynthese bevorzugt Lösemittel eingesetzt, die ganz oder teilweise wasserverdünnbar sind. Sollen das Verfahrensprodukt zum Beispiel dort eingesetzt werden, wo die Anwesenheit von flüchtigen organischen Verbindungen (VOC) nicht erwünscht ist, so sollte die Formulierung möglichst lösemittelfrei oder in entsprechend als VOC-frei geltenden Trägermaterialien vorliegen.

Je nach Anwendungsgebiet können die zur Synthese verwendeten Lösemittel im Reaktionsgemisch verbleiben oder werden ganz oder teilweise entfernt und gegebenenfalls durch andere Lösemittel oder Trägermedien ersetzt werden.
Das Lösemittel kann zum Beispiel durch Abdestillieren, gegebenenfalls bei reduziertem Druck und/oder azeotrop unter Zusatz von Wasser, ganz oder teilweise entfernt werden. Die Wirksubstanz (Verbindung der allgemeinen Formel (I)) kann aber auch durch Ausfällen mittels Zusatz von Nichtlösern wie aliphatischen Kohlenwasserstoffen, beispielsweise Hexan, und anschließendem Abtrennen durch Filtration und gegebenenfalls Trocknen isoliert werden. Die nach einer dieser Methoden erhaltene Wirksubstanz kann dann in einem für das jeweilige Anwendungsgebiet geeigneten Lösemittel angelöst werden oder gegebenenfalls in reiner Form beispielsweise bei Pulverlacken, eingesetzt werden oder auf inerte Träger aufgezogen werden. Für Anwendungen, bei denen die Verwendung von Feststoffen bevorzugt ist, wie Pulverlacken oder bestimmten Kunststoffverarbeitungsverfahren, können die Verbindungen auch durch weitere bekannte Verfahren in eine feste Form überführt werden. Beispiele für solche Verfahren sind Mikroverkapselung, Sprühtrocknung, Adsorption auf einen festen Träger wie SiO₂ oder das PGSS-Verfahren (Particle from Gas Saturated Solutions).

### Modifikationen

In einer weiteren Ausführungsform können die im erfindungsgemäßen amidoamingruppenhaltigen Reaktionsprodukt noch vorhandenen XH-Gruppen, das heißt OH-Gruppen und/oder primäre und/oder sekundäre Aminogruppen in einer Folgereaktion weiter umgesetzt werden, zum Beispiel mit Carbonsäureanhydriden. Die erfindungsgemäßen amidoamingruppenhaltigen Reaktionsprodukte dienen dann als Zwischenprodukte bei der Herstellung modifizierter ebenfalls erfindungsgemäßer amidoamingruppenhaltiger Reaktionsprodukte. Die modifizierten Produkte können auf den gleichen Gebieten eingesetzt werden wie die noch nicht modifizierten erfindungsgemäßen amidoamingruppenhaltigen Reaktionsprodukte. Durch die Modifizierung kann beispielsweise die Verträglichkeit der Reaktionsprodukte gegenüber bestimmten Medien erhöht werden oder angepasst werden.

Die tertiären Aminogruppen können auch mit Sauerstoff, Peroxoverbindungen wie Percarbonsäuren und mit Wasserstoffperoxid in **Aminoxide** überführt werden, die zusätzlich mit Säuren wie zum Beispiel Salzsäure versalzt werden können.

### Versalzungsprodukte

Die erfindungsgemäßen amidoamingruppenhaltigen Reaktionsprodukte beziehungsweise die darin enthaltenen Spezies der allgemeinen Formel (I) enthalten tertiäre Aminogruppen. Diese können ganz oder teilweise versalzt werden. Die tertiären Aminogruppen können beispielsweise mit Säuren, wie Carbonsäuren, Carbonsäurederivaten wie z.B. Carbonsäurehalogeniden oder Phosphorsäuren und deren Estern, zu entsprechenden Ammoniumsalzen umgesetzt werden. Die erfindungsgemäßen amidoamingruppenhaltigen Reaktionsprodukte dienen dann als Zwischenprodukte bei der Herstellung versalzter ebenfalls erfindungsgemäßer amidoamingruppenhaltiger Reaktionsprodukte. Die versalzten Produkte können auf den gleichen Gebieten eingesetzt werden wie die nicht versalzten erfindungsgemäßen amidoamingruppenhaltigen Reaktionsprodukte. Durch die Versalzung kann beispielsweise die Verträglichkeit der Reaktionsprodukte gegenüber bestimmten Medien erhöht werden oder angepasst werden oder die Wechselwirkung mit Feststoffpartikeln wie Pigmenten und/oder Füllstoffen beeinflusst werden.

### Quaternisierungsprodukte

Die erfindungsgemäßen amidoamingruppenhaltigen Reaktionsprodukte beziehungsweise die darin enthaltenen Spezies der allgemeinen Formel (I) enthalten tertiäre Aminogruppen. Diese können ganz oder teilweise durch Umsetzung mit Quaternisierungsreagenzien in entsprechende **quartäre Ammoniumsalze** überführt werden. Die erfindungsgemäßen amidoamingruppenhaltigen Reaktionsprodukte dienen dann als Zwischenprodukte bei der Herstellung quaternisierter ebenfalls erfindungsgemäßer amidoamingruppenhaltiger Reaktionsprodukte. Die quaternisierten Produkte können auf den gleichen Gebieten eingesetzt werden wie die nicht quaternisierten erfindungsgemäßen amidoamingruppenhaltigen Reaktionsprodukte. Durch die Quaternisierung kann beispielsweise die Verträglichkeit der Reaktionsprodukte gegenüber bestimmten Medien erhöht werden oder angepasst werden oder die Wechselwirkung mit Feststoffpartikeln wie Pigmenten und/oder Füllstoffen beeinflusst werden.

Geeignete Quaternisierungsreagenzien können zum Beispiel gewählt werden aus der Gruppe der Alkylhalogenide und Aralkylhalogenide oder Aralkylverbindungen mit Abgangsgruppen wie Triflat, Methylsulfat oder Tosylat die nukleophile Substitutionsreaktionen mit tertiären Aminen eingehen können oder Oxiranen, wie Alkylenoxiden oder Glycidethern, in Gegenwart von Säuren oder deren Derivaten, wie Carbonsäuren, Sulfonsäuren oder Phosphorsäuren und deren Estern oder Halogeniden.

Beispiele für geeignete Quaternisierungsreagenzien sind Benzylchlorid, 2- oder 4-Vinylbenzylchlorid, Methylchlorid, Methyljodid, Methyltosylat oder Dimethylsulfat. Bevorzugt sind Benzylchlorid und 4-Vinylbenzylchlorid.

Eine weitere Möglichkeit zur Quaternisierung ist die Verwendung von Glycidylethern in Gegenwart von Säuren. Beispiele für geeignete Gycidylether sind Glycidylmethacrylat, Alkylglycidylether wie 2-Ethylhexylglycidylether und C13/C15-Glycidylether (Handelsname z.B. Grilonit RV 1814) oder Arylglycidylether wie Kresylglycidylether. Für diese Quaternisierungsreaktion geeignete Säuren sind zum Beispiel Carbonsäuren wie Benzoesäure, Essigsäure oder Milchsäure. Weitere Säuren sind saure Phosphorsäureester mit einer oder zwei Estergruppen.

### Erfindungsgemäße Verwendung

Die vorliegende Erfindung betrifft weiterhin die Verwendung der vorstehend beschriebenen erfindungsgemäßen amidoamingruppenhaltigen Reaktionsprodukte oder der darin enthaltenen Spezies der allgemeinen Formel (I) als Additiv, bevorzugt als Netzmittel und/oder Dispergiermittel und/oder Dispersionsstabilisator in Zusammensetzungen, wie Feststoffgemengen oder Beschichtungen, insbesondere Lacken, Kunststoffen, Pigmentpasten, Dichtstoffen, Kosmetika, Keramik, Klebstoffen, Vergussmassen, Spachtelmassen, Druckfarben und Tinten.

Außerdem betrifft die Erfindung auch die oben aufgeführten Zusammensetzungen, wie Feststoffgemengen oder Beschichtungen, insbesondere Lacken, Kunststoffen, Pigmentpasten, Dichtstoffen, Kosmetika, Keramik, Klebstoffen, Vergussmassen, Spachtelmassen, Druckfarben und Tinten. Die Feststoffgemenge enthalten Partikel und/oder Fasern, die mit den vorstehend beschriebenen erfindungsgemäßen amidoamingruppenhaltigen Reaktionsprodukten oder den darin enthaltenen Spezies der allgemeinen Formel (I) behandelt worden sind.

Die erfindungsgemäßen amidoamingruppenhaltigen Reaktionsprodukte oder die darin enthaltenen Spezies der allgemeinen Formel (I) finden beispielsweise Verwendung als Aluminiumpassivatoren, Dispergiermittel, Dispersionsstabilisatoren oder Netzmittel und lassen sich beispielsweise in pigmentierten und/oder füllstoffhaltigen Produkten, beispielsweise Pigmentkonzentraten oder -pasten, Beschichtungszusammensetzungen, Dichtstoffen, Kunststoffen, Keramiken, Kosmetika, Klebstoffen, Vergussmassen, Spachtelmassen, Druckfarben und/oder Tinten einsetzen. Bevorzugt sind Pigmentkonzentrate, die mit entsprechenden Auflacksystemen gemischt werden können, wodurch pigmentierte Lacke hergestellt werden.

So können die erfindungsgemäßen amidoamingruppenhaltige Reaktionsprodukte oder die darin enthaltenen Spezies der allgemeinen Formel (I) beispielsweise bei der Herstellung oder Verarbeitung von Lacken, Druckfarben, Tinten, beispielsweise für Tintenstrahldruck, Papierstrich, Leder- und Textilfarben, Pasten, Pigmentkonzentraten, Keramiken, Kleb- und Dichtstoffen, Vergussmassen, Kunststoffen und kosmetischen Zubereitungen eingesetzt werden, insbesondere, wenn diese Feststoffe wie Pigmente und/oder Füllstoffe (auch faserförmige) enthalten.

Auch bei der Herstellung oder Verarbeitung von **Formmassen** auf Basis von synthetischen, halbsynthetischen oder natürlichen makromolekularen Stoffen, wie Polyvinylchlorid, gesättigten oder ungesättigten Polyestern, Polyurethanen, Polystyrolen, Polyacrylaten, Polyamiden, Epoxidharzen, Polyolefinen, wie Polyethylen oder Polypropylen, können diese eingesetzt werden. Beispielsweise lassen sich diese zur Herstellung von Vergussmassen, Gießmassen, PVC-Plastisolen, Gelcoats, Polymerbeton, Leiterplatten, Industrielacken, Holz- und Möbellacken, Fahrzeuglacken, Schiffsfarben, Korrosionsschutzfarben, Can- und Coil-Coatings oder Maler- und Bautenlacken verwenden.

Die erfindungsgemäßen amidoamingruppenhaltigen Reaktionsprodukte oder die darin enthaltenen Spezies der allgemeinen Formel (I) können nicht nur in Auflacksystemen für pigmentierte Lacke eingesetzt werden. Ebenfalls möglich ist der Einsatz in einem weiten Bereich von Formulierungen bzw. Produkten, wie Harzen, Ölen, Fetten, Gleitmitteln, Gummimaterialien, Dichtstoffen, Druckfarben, Tinten, Klebstoffen, Wachsen oder Beschichtungsmittelzusammensetzungen. Die Konzentrate können auch eingesetzt werden in Formulierungen, die in der Körperpflegeindustrie hergestellt werden oder in elektrischen Anwendungen in der Elektronikindustrie, in der Schiffsindustrie, im Rahmen medizinischer Anwendungen, in der Bauindustrie oder in der Automobilindustrie. Beispiele schließen elektronisches Papier, wie das Display bei E-Books, die Verkapselung mikroelektronischer Chips und Leiterplatten, Unterwasserbootshautbeschichtungen, wie Anti-Fouling-Beschichtungen, Silikonröhren oder Gleitadditive für Bremskomponenten ein.

Die erfindungsgemäßen amidoamingruppenhaltige Reaktionsprodukte oder die darin enthaltenen Spezies der allgemeinen Formel (I) können vorteilhaft auch bei der Herstellung von **Farbfiltern** für Flüssigkristallanzeigen, Flüssigkristallbildschirme, Farbauflösungsgeräte, Sensoren, Plasmabildschirmen, Anzeigen auf Basis der SED (Surface conduction Electron emitter Display) und für **MLCC** (Multi Layer Ceramic Compounds) eingesetzt werden. Die MLCC-Technologie wird bei der Herstellung von Mikrochips und Leiterplatten angewendet.

Die Verwendung **in kosmetischen Präparaten** kann beispielsweise zur Herstellung kosmetischer Zubereitungen wie Make-up, Puder, Lippenstifte, Haarfärbemittel, Cremes, Nagellacke und Sonnenschutzpräparaten dienen. Diese können in den üblichen Formen, beispielsweise als W/O- oder O/W-Emulsionen, Lösungen, Gele, Cremes, Lotionen oder Sprays vorliegen. Die erfindungsgemäßen amidoamingruppenhaltige Reaktionsprodukte oder die darin enthaltenen Spezies der allgemeinen Formel (I) können vorteilhaft in zur Herstellung dieser Zubereitungen verwendeten Dispersionen eingesetzt werden. Diese können die für diese Zwecke in der Kosmetik üblichen Trägermedien, wie Wasser, Ricinusöle oder Silikonöle und Feststoffe, wie organische und anorganische Pigmente, wie Titandioxid oder Eisenoxid, enthalten. Zu nennen sind ebenfalls die Anwendungsbereiche NIP (Non impact printing), InkJet (auf Papier, Folie, Keramik, künstlichem und natürlichem Fasergewebe), Dispergieren von Keramik (wässrig oder wasserfrei), Dispergieren in Vergussmassen. Die erfindungsgemäßen amidoamingruppenhaltige Reaktionsprodukte oder die darin enthaltenen Spezies der allgemeinen Formel (I) können auch als solche, d.h. ohne zuvor in ein entsprechendes Konzentrat eingearbeitet worden zu sein, in den zuvor genannten Formulierungen und Anwendungsbereichen zum Einsatz kommen.

Typischerweise ist das Produkt, welches die erfindungsgemäßen amidoamingruppenhaltigen Reaktionsprodukte oder die darin enthaltenen Spezies der allgemeinen Formel (I) sowie Pigment- und/oder Füllstoffe enthält, ein Lack, oder ein Pigmentkonzentrat für Beschichtungszusammensetzungen. Letztlich aber ist der Einsatz der erfindungsgemäßen amidoamingruppenhaltige Reaktionsprodukte oder der darin enthaltenen Spezies der allgemeinen Formel (I) in beliebigen pigmenthaltigen und/oder füllstoffhaltigen Produkten möglich.

Insbesondere handelt es sich bei den **Pigmentkonzentraten** um Zusammensetzungen, die neben der erfindungsgemäßen amidoamingruppenhaltigen Reaktionsprodukten oder den darin enthaltenen Spezies der allgemeinen Formel (I), beispielsweise organische Lösemittel und mindestens ein Pigment enthalten. Diese enthalten insbesondere keine oder nur geringe Anteile organischer Polymere als Bindemittel. Solche bekannten Bindemittel sind vorteilhafterweise in den entsprechenden Auflacksystemen vorhanden und werden nachstehend beschrieben.

Als organische **Lösemittel** kommen insbesondere die typischen im Bereich der Lack- und Farbindustrie eingesetzten, dem Fachmann bekannten organischen Lösemittel, wie aliphatische Lösemittel, cycloaliphatische Lösemittel, aromatische Lösemittel, wie Toluol, Xylol, Solventnaphtha, Ether, Ester und/oder Ketone, beispielsweise Butylglykol, Butyldiglykol, Butylacetat, Methylisobutylketon, Methylethylketon und/oder Lösemittel, wie Methoxypropylacetat, Diacetonalkohol, zum Einsatz.

Als **Pigmente** kommen die dem Fachmann bekannten Pigmente zu Einsatz. Häufig werden Kombinationen verschiedener Pigmente eingesetzt um die gewünschten Eigenschaften zu erhalten. Beispiele für Pigmente sind Mono-, Di-, Tri- und Polyazopigmente, Oxazin-, Dioxazin-, Thiazin-Pigmente, Diketo-pyrrolo-pyrrole, Phthalocyanine, Ultramarin und andere Metallkomplex-Pigmente, indigoide Pigmente, Diphenylmethan-Pigmente, Triarylmethan-Pigmente, Xanthen-Pigmente, Acridin-Pigmente, Chinacridon-Pigmente, Methin-Pigmente, Anthrachinon, Pyranthron-, Perylen-Pigmente und andere polycyclische Carbonylpigmente, anorganische Pigmente, wie Carbon Black Pigmente und/oder Pigmente auf Basis von Russ, Graphit, Zink, Titandioxid, Zinkoxid, Zinksulfid, Zinkphosphat, Bariumsulfat, Lithophone, Eisenoxid, Ultramarin, Manganphosphat, Cobaltaluminat, Cobaltstannat, Cobaltzinkat, Antimonoxid, Antimonsulfid, Chromoxid, Zinkchromat, gemischte Metalloxide auf Basis von Nickel, Bismut, Vanadium, Molybdän, Cadmium, Titan, Zink, Mangan, Cobalt, Eisen, Chrom, Antimon, Magnesium, Aluminium (beispielsweise Nickeltitangelb, Bismut-vanadat-molybdatgelb oder Chromtitangelb), magnetische Pigmente auf Basis von Reineisen, Eisenoxiden und Chromoxiden oder Mischoxiden, Metalleffektpigmente aus Aluminium, Zink, Kupfer oder Messing sowie Perlglanzpigmente oder fluoreszierende und phosphoreszierende Leuchtpigmente. Weitere Beispiele sind nanoskalige organische oder anorganische Feststoffe mit Teilchengrößen unterhalb von 100 nm in mindestens einer Dimension, wie bestimmte Russtypen oder andere allotrope Formen des Kohlenstoffs, wie single-wall-CNT's, multi-wall-CNT's und Graphen. Die Bestimmung der Teilchengröße erfolgt beispielsweise mittels Transmissionselektronenmikroskopie, analytischer Ultrazentrifugation oder Methoden der Lichtstreuung. Ebenfalls zu nennen sind Partikel, die aus einem Metall-, oder Halbmetalloxid bzw. -hydroxid bestehen, sowie Partikel, die aus gemischten Metall- und/oder Halbmetalloxiden bzw. -hydroxiden bestehen. Beispielsweise lassen sich die Oxide und/oder Oxidhydroxide des Aluminiums, Siliziums, Zinks, Titans, usw. zur Herstellung solcher extrem feinteiligen Feststoffe heranziehen. Der Herstellprozess dieser oxidischen bzw. hydroxidischen bzw. oxidhydroxidischen Teilchen kann über die unterschiedlichsten Verfahren wie beispielsweise Ionenaustauschprozesse, Plasmaprozesse, Sol-Gel-Verfahren, Ausfällung, Zerkleinerung (beispielsweise durch Vermahlung) oder Flammhydrolyse erfolgen. Alle vorstehend genannten Pigmente können oberflächenmodifiziert vorliegen und basische, saure oder neutrale Gruppen an der Oberfläche besitzen.

Weitere Beispiele sind die folgenden unter ihrer Color Index Nummer (C.I.) aufgeführten Pigmente:
Beispiele für Rotpigmente sind C. I. Pigment Red 1, 2, 3, 4, 5, 6, 7, 8, 9, 12, 14, 15, 16, 17, 21, 22, 23, 31, 32, 37, 38, 41, 47, 48, 48:1, 48:2, 48:3, 48:4, 49, 49:1, 49:2, 50:1, 52:1, 52:2, 53, 53:1, 53:2, 53:3, 57, 57:1, 57:2, 58:4, 60, 63, 63:1, 63:2, 64, 64:1, 68, 69, 81, 81:1, 81:2, 81:3 ,81:4, 83, 88, 90:1, 101, 101:1, 104 ,108, 108:1, 109, 112, 113, 114, 122, 123, 144, 146, 147, 149, 151, 166, 168, 169, 170, 172, 173, 174, 175, 176, 177, 178, 179, 181, 184, 185, 187, 188, 190, 193, 194, 200, 202, 206, 207, 208, 209, 210, 214, 216, 220, 221, 224, 230, 231, 232, 233, 235. 236, 237, 238, 239, 242, 243, 245, 247, 249, 250, 251, 253, 254, 255, 256, 257, 258, 259, 260, 262, 263, 264, 265, 266, 267, 268, 269, 270, 271, 272, 273, 274, 275, und 276.
Beispiele für Blaupigmente sind C. I. Pigment Blue 1, 1:2, 9, 14, 15, 15:1, 15:2, 15:3, 15:4, 15:6, 16, 17, 19, 25, 27, 28, 29, 33, 35, 36, 56, 56:1, 60, 61, 61:1, 62, 63, 66, 67, 68, 71, 72, 73, 74, 75, 76, 78,und 79.
Beispiele für Grünpigmente sind C. I. Pigment Green 1, 2, 4, 7, 8, 10, 13, 14, 15, 17, 18, 19, 26, 36, 45, 48, 50, 51, 54, 55, 58 oder 59.
Beispiele für Gelbpigmente sind C. I. Pigment Yellow 1, 1:1, 2, 3, 4, 5, 6, 9, 10, 12, 13, 14, 16, 17, 24, 31, 32, 34, 35, 35:1, 36, 36:1, 37, 37:1, 40, 41, 42, 43, 48, 53, 55, 61, 62, 62:1, 63, 65, 73, 74, 75, 81, 83, 87, 93, 94, 95, 97, 100, 101, 104, 105, 108, 109, 110, 111, 116, 117, 119, 120, 126, 127, 127:1, 128, 129, 133, 134, 136, 138, 139, 142, 147, 148, 150, 151, 153, 154, 155, 157, 158, 159, 160, 161, 162, 163, 164, 165, 166, 167, 168, 169, 170, 172, 173, 174, 175, 176, 180, 181, 182, 183, 184, 185, 188, 189, 190, 191, 191:1, 192, 193, 194, 195, 196, 197, 198, 199, 200, 202, 203, 204, 205, 206, 207, und 208.
Beispiele für Violettpigmente sind C. I. Pigment Violet 1, 1:1, 2, 2:2, 3, 3:1, 3:3, 5, 5:1, 14, 15, 16, 19, 23, 25, 27, 29, 31, 32, 37, 39, 42, 44, 47, 49, und 50.
Beispiele für Orangepigmente sind C. I. Pigment Orange 1, 2, 5, 13, 16, 17, 19, 20, 21, 22, 23, 24, 34, 36, 38, 39, 43, 46, 48, 49, 61, 62, 64, 65, 67, 68, 69, 70, 71, 72, 73, 74, 75, 77, 78, und 79.
Beispiele für Schwarzpigmente sind C. I. Pigment Black 7, 11, 30, 33.

Enthalten die jeweiligen Produkte, insbesondere die Beschichtungszusammensetzungen, **Füllstoffe,** so handelt es sich zum Beispiel um die dem Fachmann bekannten Füllstoffe. Beispiele für pulver- oder faserförmige Füllstoffe sind zum Beispiel solche, die aufgebaut sind aus pulver- oder faserförmigen Teilchen von Aluminiumoxid, Aluminiumhydroxid, Siliziumdioxid, Kieselgur, Kieselerde, Quarz, Kieselgel, Talkum, Kaolin, Glimmer, Perlite, Feldspat, Schiefermehl, Calciumsulfat, Bariumsulfat, Calciumcarbonat, Calcit, Dolomit, Glas oder Kohlenstoff. Die eingesetzten Fasern können organischer und/oder anorganischer Natur sein und ebenso als Verstärkungsstoffe eingesetzt werden. Weitere Beispiele für Pigmente oder Füllstoffe finden sich beispielsweise in der US-A-4,795,796. Auch Flammschutzmittel, sofern die erfindungsgemäßen Verbindungen nicht bereits in üblichen Additivmengen für diesen Zweck eingesetzt werden, wie Aluminium- oder Magnesiumhydroxid und Mattierungsmittel, wie Kieselsäuren, lassen sich ebenfalls besonders gut durch die erfindungsgemäßen amidoamingruppenhaltigen Reaktionsprodukte oder die darin enthaltenen Spezies der allgemeinen Formel (I) dispergieren und stabilisieren.

Die erfindungsgemäßen amidoamingruppenhaltigen Reaktionsprodukte oder die darin enthaltenen Spezies der allgemeinen Formel (I) eignen sich insbesondere auch zur Herstellung von **Feststoffkonzentraten, wie Pigmentkonzentraten.** Dazu werden die erfindungsgemäßen amidoamingruppenhaltigen Reaktionsprodukte oder die darin enthaltenen Spezies der allgemeinen Formel (I) in einem Trägermedium wie organischen Lösemitteln, Weichmachern und/oder Wasser vorgelegt und die zu dispergierenden Feststoffe werden unter Rühren zugegeben. Zusätzlich können diese Konzentrate Bindemittel und/oder andere Hilfsstoffe enthalten. Mit den erfindungsgemäßen amidoamingruppenhaltigen Reaktionsprodukten oder den darin enthaltenen Spezies der allgemeinen Formel (I) ist es aber insbesondere möglich, stabile bindemittelfreie Pigmentkonzentrate herzustellen. Ebenso ist es möglich, mit den erfindungsgemäßen amidoamingruppenhaltigen Reaktionsprodukten oder den darin enthaltenen Spezies der allgemeinen Formel (I) fließfähige Feststoffkonzentrate aus Pigmentpresskuchen herzustellen. Hierbei wird dem Presskuchen, der noch organische Lösemittel, Weichmacher und/oder Wasser enthalten kann, die erfindungsgemäßen amidoamingruppenhaltigen Reaktionsprodukte oder die darin enthaltenen Spezies der allgemeinen Formel (I) zugemischt und die so erhaltene Mischung dispergiert. Die auf verschiedenen Wegen hergestellten Feststoffkonzentrate können dann in unterschiedliche Substrate wie z.B. Alkydharze, Polyesterharze, Acrylatharze, Polyurethanharze oder Epoxidharze eingearbeitet werden. Pigmente können aber auch lösemittelfrei direkt in den erfindungsgemäßen amidoamingruppenhaltigen Reaktionsprodukte oder den darin enthaltenen Spezies der allgemeinen Formel (I) dispergiert werden und eignen sich dann besonders zur Pigmentierung von thermoplastischen und duroplastischen Kunststoffformulierungen.

### Einsatzmengen

Je nach Einsatzgebiet werden die erfindungsgemäßen amidoamingruppenhaltigen Reaktionsprodukte oder die darin enthaltenen Spezies der allgemeinen Formel (I) in solchen Mengen eingesetzt, dass in dem letztlich für die weitere Anwendung interessanten Produkt vorzugsweise ein Anteil des erfindungsgemäßen Netz- und Dispergiermittels, der erfindungsgemäßen amidoamingruppenhaltigen Reaktionsprodukte oder der darin enthaltenen Spezies der allgemeinen Formel (I) von 0,01 bis 10 Gew.-%, bezogen auf die Gesamtmenge des jeweiligen Produkts, besteht. Möglich sind aber auch höhere Anteile.

Bezogen auf den zu dispergierenden Feststoff, beispielsweise das Pigment, werden die erfindungsgemäßen amidoamingruppenhaltigen Reaktionsprodukte oder die darin enthaltenen Spezies der allgemeinen Formel (I) in einer Menge von bevorzugt 0,5 bis 100 Gew.-% eingesetzt. Werden schwer zu dispergierende Feststoffe verwendet, kann die Menge an eingesetztem erfindungsgemäßen Netz- und Dispergiermittel, den erfindungsgemäßen amidoamingruppenhaltigen Reaktionsprodukten oder den darin enthaltenen Spezies der allgemeinen Formel (I) durchaus höher sein. Die Menge ist im Allgemeinen abhängig von der zu belegenden Oberfläche des zu dispergierenden Stoffes. Von Bedeutung kann also beispielsweise sein, um welches Pigment es sich handelt. Allgemein lässt sich sagen, dass zur Dispergierung anorganischer Pigmente meist weniger Dispergiermittel notwendig ist als für organische Pigmente, da letztere meist über eine höhere spezifische Oberfläche verfügen und von daher eine größere Menge an Dispergiermittel nötig ist. Typische Dosierungen des Netz- und Dispergiermittels bzw. der erfindungsgemäßen amidoamingruppenhaltigen Reaktionsprodukte oder der darin enthaltenen Spezies der allgemeinen Formel (I) sind für anorganische Pigmente beispielsweise 1 bis 20 Gew.-% und für organische Pigmente 10 bis 50 Gew.-%, jeweils bezogen auf den zu dispergierenden Feststoff, insbesondere das Pigment. Bei sehr feinteiligen Pigmenten (beispielsweise einigen Rußen) können auch Zugabemengen von 30 bis 90% oder mehr sinnvoll sein. Als Kriterien für eine ausreichende Pigmentstabilisierung können beispielsweise Glanz und Transparenz der Beschichtungszusammensetzungen oder der Grad des Ausschwimmens herangezogen werden. Die Dispergierung der Feststoffe kann als **Einzelanreibung** erfolgen oder auch als **Gemischanreibung** mit mehreren Pigmenten gleichzeitig, wobei die besten Ergebnisse in der Regel bei Einzelanreibungen zu erreichen sind. Bei der Verwendung von Mischungen verschiedener Feststoffe kann es durch gegensätzliche Ladungen auf den Feststoffoberflächen verstärkt zu Agglomerationen in der flüssigen Phase kommen. In diesen Fällen lässt sich bei Einsatz der erfindungsgemäßen amidoamingruppenhaltigen Reaktionsprodukte oder der darin enthaltenen Spezies der allgemeinen Formel (I) häufig eine gleichnamige, in der Regel positive, Ladung aller Teilchen erreichen und damit Instabilitäten durch Ladungsunterschiede vermeiden. Ihre optimale Wirkung erzielen die Dispergiermittel bzw. die erfindungsgemäßen amidoamingruppenhaltigen Reaktionsprodukte oder die darin enthaltenen Spezies der allgemeinen Formel (I) bei Zugabe zum Mahlgut, insbesondere, wenn zunächst der zu dispergierende Feststoff nur mit dem Additiv und gegebenenfalls Lösemitteln gemischt wird ("Premix"), da dann das Additiv bevorzugt auf die Feststoffoberfläche adsorbieren kann, ohne in Wettbewerb mit den Bindemittel-Polymeren treten zu müssen. In der Praxis ist diese Vorgehensweise aber nur in Ausnahmefällen nötig. Bei Bedarf können die erfindungsgemäßen amidoamingruppenhaltigen Reaktionsprodukte oder die darin enthaltenen Spezies der allgemeinen Formel (I) auch nachträglich eingesetzt werden (als sogenannte "Post-Additive"), zum Beispiel, um in einem fertig aufgelackten Ansatz Ausschwimm- oder Flockulationsprobleme zu lösen. In der Regel sind in diesem Fall aber erhöhte Additiv-Dosierungen erforderlich.

Die Produkte, insbesondere die Beschichtungszusammensetzungen beziehungsweise Lacke, in denen die erfindungsgemäßen amidoamingruppenhaltigen Reaktionsprodukte oder die darin enthaltenen Spezies der allgemeinen Formel (I) letztlich ihre Wirkungen entfalten sollen, können zudem ein organisches Polymer als weiteres **Bindemittel** enthalten. Solche Bindemittel sind dem Fachmann bekannt. Dieses mindestens eine weitere Bindemittel kann beispielsweise über ein Auflacksystem eingeführt werden, welches beispielsweise mit einem Pigmentkonzentrat, enthaltend die erfindungsgemäßen amidoamingruppenhaltigen Reaktionsprodukte oder die darin enthaltenen Spezies der allgemeinen Formel (I), gemischt wird, so dass es sich bei dem betrachteten Produkt um einen pigmentierten Lack handelt. Möglich sind aber auch andere pigmentierte und/oder füllstoffhaltige Produkte, beispielsweise Kunststoffe, Dichtstoffe und weitere dem Fachmann bekannte Produkte auf Basis einer organischen polymeren Matrix. Als Produkt ist ein System anzusehen, welches ein polymeres Harz beziehungsweise organisches Polymer als Bindemittel enthält und dadurch in der Lage ist, unter geeigneten Härtungsbedingungen eine feste organische, polymere Matrix zu bilden (beispielsweise eine Beschichtungszusammensetzung). Ebenfalls als Produkt wird ein System bezeichnet, dass durch einfache Vermischung mit einer Komponente, welche ein Bindemittel enthält, zur Bildung einer solchen organischen, polymeren Matrix fähig ist (beispielsweise ein Pigmentkonzentrat). Zum Einsatz kommen beispielsweise, aber nicht ausschließlich die dem Fachmann bekannten Alkydharze, Polyesterharze, Acrylatharze, Polyurethanharze, Cellulosenitrate, Celluloseacetobutyrate, Melamine, Chlorkautschuken und/oder Epoxidharze. Beispiele für Beschichtungen auf Wasserbasis sind kathodische oder anodische Elektrotauchlackierungen z.B. für Automobilkarossen. Weitere Beispiele sind Putze, Silikatfarben, Dispersionsfarben, Wasserlacke auf Basis von wasserverdünnbaren Alkyden, Alkydemulsionen, Hybridsystemen, 2-Komponenten-Systemen, Polyurethan- und Acrylat-Dispersionen.

Möglich sind sowohl 1-Komponentensysteme als auch 2-Komponentensysteme, wobei im letzteren Fall in der Regel noch Polyisocyanate, Melaminharze und/oder Polyamidharze als die typischen, dem Fachmann geläufigen Vernetzungsmittel in einer zweiten Komponente vorhanden sind. Bevorzugt sind Produktssysteme, insbesondere Beschichtungszusammensetzungen, die eine Acrylatharz als Bindemittel enthalten. In einer weiteren Variante handelt es sich um eine 2-Komponenten (2K) Beschichtungszusammensetzung bzw. einen 2K-Lack, der ein Epoxidharz in der Bindemittelkomponente und ein Polyamidharz in der Vernetzerkomponente enthält.

Die als Produkte bevorzugten Beschichtungszusammensetzungen können **wasserbasierend oder lösemittelbasierend** sein. Unter wasserbasierend ist zu verstehen, dass die Beschichtungszusammensetzung als Lösemittel hauptsächlich Wasser enthält. Insbesondere sind bei einer wasserbasierenden Beschichtungszusammensetzung vorzugsweise nicht mehr als 10 Gew.-% organische Lösemittel, bezogen auf die Gesamtmenge an Lösemitteln, in der Beschichtungszusammensetzung enthalten. Als lösemittelbasierend gilt eine Beschichtungszusammensetzung, die nicht mehr als 5 Gew.-%, bevorzugt nicht mehr als 2 Gew.-% Wasser, bezogen auf die Gesamtmenge an Lösemitteln enthält.

Als **weitere Produktkomponenten** kommen beispielsweise in Frage Photoinitiatoren, Entschäumer, Netzmittel, filmbildende Hilfsmittel, wie Cellulose-Derivate (beispielsweise Cellulosenitrate, Celluloseacetate, Celluloseacetobutyrat), Reaktivverdünner, Verlaufmittel, Dispergiermittel, und/oder rheologiesteuernde Additive.

Die **Herstellung der als Produkte bevorzugten Pigmentkonzentrate** und Beschichtungszusammensetzung erfolgt über die dem Fachmann geläufigen Verfahren. Es kommen die bekannten Methoden zum Einsatz, wie beispielsweise die schrittweise Zugabe unter Rühren und Vermischung der Bestandteile der Beschichtungszusammensetzung in üblichen Mischaggregaten, wie Rührkesseln oder Dissolvern.

Unter Verwendung der bevorzugten Pigmentkonzentrate und Beschichtungszusammensetzungen können Beschichtungen bzw. Lackschichten hergestellt werden. Die Herstellung der Beschichtung erfolgt über die dem Fachmann geläufigen Applikationstechniken auf ein Substrat und anschließende Härtungsverfahren.

Die **Applikation** erfolgt beispielsweise durch die bekannten Spritz-, Sprüh-, Streich-, Roll-, Giess-, Tränk- und/oder Tauchverfahren. Nach der Applikation der Beschichtungszusammensetzung auf ein Substrat erfolgt die Härtung beziehungsweise Trocknung nach gängigen Methoden. Z.B. kann die applizierte Beschichtungszusammensetzung physikalisch trocknend, thermisch und/oder unter Anwendung aktinischer Strahlung (strahlenhärtend), vorzugsweise UV-Strahlung sowie Elektronenstrahlung härtbar sein. Die thermische Härtung kann beispielsweise im Bereich von etwa 10°C bis etwa 400 °C, je nach Art der Beschichtungszusammensetzung und/oder des Substrats, erfolgen. Auch die Dauer der Härtung ist individuell beispielsweise von der Art des Härtungsverfahrens (thermisch oder aktinisch), der Art der eingesetzten Beschichtungszusammensetzung und/oder den Substraten abhängig. Dabei kann das Substrat bewegt werden oder auch ruhen.

Neben der vorstehend beschriebenen Anwendung als Dispergiermittel und/oder Beschichtungsmittel für pulver- und faserförmige Feststoffe können die erfindungsgemäßen amidoamingruppenhaltigen Reaktionsprodukte oder die darin enthaltenen Spezies der allgemeinen Formel (I) auch als **Viskositätsreduzierer und Kompatibilisatoren in Kunstharzen** eingesetzt werden. Beispiele für solche Kunstharze sind die sogenannten "sheet molding compounds" (SMC) und "bulk molding compounds" (BMC), die aus stark füllstoff- und faserhaltigen ungesättigten Polyesterharzen bestehen. Ihre Herstellung und Verarbeitung ist beispielhaft in der US-A-4,777,195 beschrieben. Ein Problem bei SMC- und BMC-Kunstharzmischungen besteht darin, dass oftmals Polystyrol (PS) der Formulierung zugesetzt wird, um die Schrumpfung während des Verarbeitungsprozesses zu reduzieren. PS ist nicht mit den verwendeten ungesättigten Polyesterharzen verträglich und es kommt zur Separation der Komponenten. Bei Verwendung von PS-gefüllten SMC- oder BMC-Mischungen können die erfindungsgemäßen amidoamingruppenhaltigen Reaktionsprodukte oder die darin enthaltenen Spezies der allgemeinen Formel (I) aufgrund ihrer guten Dispergierqualitäten eine Kompatibilisierung zwischen PS und ungesättigtem Polyesterharz herbeiführen, wodurch sich die Lagerstabilität und Verarbeitungssicherheit solcher Mischungen erhöht.

Mittels der erfindungsgemäßen amidoamingruppenhaltigen Reaktionsprodukte oder die darin enthaltenen Spezies der allgemeinen Formel (I) können z.B. in unverträglichen Polyol-Mischungen, Polyol-Isocyanat-Mischungen oder Polyol-Treibmittelmischungen (werden z.B. bei der Polyurethan-Herstellung eingesetzt) **Phasenvermittlungseffekte** erzielt werden.

Im Folgenden wird die vorliegende Erfindung durch nachstehende Beispiele zusätzlich erläutert.

### BEISPIELE

Bei molekular uneinheitlichen Substanzen stellen die angegebenen Molekulargewichte - im Folgenden wie schon in der vorstehenden Beschreibung - Durchschnittswerte des Zahlenmittels dar. Die Molekulargewichte beziehungsweise zahlenmittleren Molekulargewichte Mₙ werden bei Vorhandensein titrierbarer Hydroxy- oder Amino-Gruppen durch Endgruppenbestimmung über die Ermittlung der OH-Zahl, der Amin-Zahl beziehungsweise der NCO-Zahl bestimmt.

Sofern nicht anders ausgeführt, handelt es sich bei Angaben in Teilen um Gewichtsteile und bei Angaben in Prozenten um Gewichtsprozente.

### Festkörper

Die Probe (2,0 ± 0,1 g Prüfsubstanz) wird in eine vorher getrocknete Aluminiumschale eingewogen und 10 Minuten bei 150 °C im Trockenschrank getrocknet, im Exsickator abgekühlt, und dann zurückgewogen. Der Rückstand entspricht dem Festkörperanteil.

### NCO-Zahl

Der freie NCO-Gehalt der zum Einsatz kommenden Polyisocyanate sowie der Reaktionsverlauf der NCO-Additionen wird nach EN ISO 9369 durch Abreaktion mit Butylamin und anschließende Titration des Aminüberschusses bestimmt. Diese Methoden sind auch beschrieben bei Saul Patai "The Chemistry of Cyanates and their Thioderivates", Part 1, Chapter 5, 1977.

### OH-Zahl

Die Bestimmung der OH-Zahl erfolgt nach DIN ISO 4629 durch Acetylierung mit einem Überschuss Essigsäureanhydrid. Anschließend wird das überschüssige Essigsäureanhydrid durch Wasserzugabe zu Essigsäure aufgespalten und mit ethanolischer KOH-Lösung zurücktitriert. Die OH-Zahl gibt die Menge KOH in mg an, die der bei der Acetylierung von 1g Substanz gebundenen Menge Essigsäure äquivalent ist.

### Aminzahl

Unter der Aminzahl (AZ) wird die Menge KOH in mg verstanden, die dem Aminanteil von 1g Substanz entspricht. Die Aminzahl wird nach DIN 16945 durch potentiometrische Titration mit 0,1 n Perchlorsäure in Essigsäure bestimmt.

### Ausgangsstoffe

- TDI:
- PMA: Methoxypropylacetat (Lösemittel), Hersteller: Dow Chemical Comp.
- DBTL: Hersteller: Merck
- DMAPA: N,N-Dimethylaminopropylamin, Hersteller: Huntsman Corp.
- TEA: Triethanolamin, Hersteller: BASF
- DEA: N,N-Diethanolamin, Hersteller: BASF
- EA: Ethanolamin, Hersteller: BASF
- IPA: 3-Imidazolyl-1-propylamin, Hersteller: BASF
- BDMAPA: N,N-Bis[3-(dimethylamino)propyl]amin = Jeffcat Z 130, Hersteller: Huntsman Corp.
- DMAE: N,N-Dimethylaminoethanol, Hersteller: BASF
- APDEA: N-(3-Aminopropyl)diethanolamin, TCI Deutschland GmbH
- AEE: 2-(2-Aminoethoxy)ethanol, Hersteller: BASF
- MA: Methylacrylat, Hersteller: BASF
- BA: Butylamin, Hersteller: BASF
- BzA: Benzylamin, Hersteller: BASF
- Grilonit RV 1814: C₁₃/C₁₅-Alkylglycidylether, Hersteller: EMS-Chemie

### Herstellung des Polyether-Polyesters Y-OH 1, Mn 780

350 g MPEG 350 (Methoxypolyethylenglykol, Mn 350), 434 g ε-Caprolacton und 1 g DBTL (Dibutylzinndilaurat) werden bei 160 °C zur Reaktion gebracht bis ein Festkörper von > 97 % erreicht ist.

Die OH-Zahl des Reaktionsproduktes beträgt 72 mg KOH/g.

Die weiteren als Y-OH verwendeten monohydroxyfunktionellen Polyester werden auf analoge Weise hergestellt.

### Allgemeine Herstellvorschrift der Monoaddukte M gemäß der allgemeinen Formel (III) (Tabelle 1):

In einem mit Rührer, Thermometer, Tropftrichter, Rückflusskühler und Stickstoffeinleitungsrohr versehenen Vierhalskolben werden 430 g Desmodur T100 (TDI) (ca. 100% 2,4-Toluoldiisocyanat, NCO-Gehalt = 48,8) und 7g Benzoylchlorid vorgelegt und gut durchmischt. X g der Alkoholkomponente, die wasserfrei und bei Polyethern alkalifrei ist, werden langsam zudosiert, so dass die Temperatur 55 °C nicht übersteigt. Das Gemisch wird nach der Zudosierung weitere 3 Stunden bei 55°C gerührt. Das überschüssige TDI wird mittels Dünnschichtverdampfer bei 150 °C aus dem Reaktionsgemisch entfernt. Der Rest-TDI-Gehalt beträgt < 1 %.

**Tabelle 1: Übersicht der Monoaddukte M gemäß der allgemeinen Formel (III)**

| Monoaddukt | Alkoholkomponente Y-OH | Menge X in [g] |
|---|---|---|
| **M1** | n-Butanolgestarteter PO-Polyether Mn 800, OH-Zahl: 70 mg KOH/g | 800 |
| **M2** | MPEG 350, OH-Zahl: 160 mg KOH/g | 350 |
| **M3** | n-Butanolgestarteter EO/PO-Polyether (EO:PO 1:1) Mn 2240, OH-Zahl: 25 mg KOH/g | 2240 |
| **M4** | Polyester **Y-OH 1**, OH-Zahl: 72 mg KOH/g | 780 |
| **M5** | Hexadecanolgestarteter Monohydroxyfunktioneller ε-Caprolactonpolyester, Mn 600 | 600 |
| **M6** | Hexadecanolgestarteter Monohydroxyfunktioneller ε-Caprolactonpolyester, Mn 1200 | 1200 |
| **M7** | MPEG 500 = Methoxypolyethylenglykol, Mn 500 | 500 |
| **M8** | n-Butanolgestarteter EO/PO-Polyether (EO:PO 1:1) Mn 1100 | 1100 |
| **M9** | n-Butanolgestarteter EO/PO-Polyether (EO:PO 1:1) Mn 1500 | 1500 |
| **M10** | n-Butanolgestarteter EO/PO-Polyether (EO:PO 1:1) Mn 1800 | 1800 |
| **M11** | n-Butanolgestarteter EO/PO-Polyether (EO:PO 1:1) Mn 3100 | 3100 |
| **M12** | n-Butanolgestarteter EO/PO-Polyether (EO:PO 1:1) Mn 4250 | 4250 |
| **M13** | Monohydroxyfunktionelles Hydroxypropyl-polydimethylsiloxan mit Butylendgruppe, Mn 1200 | 1200 |
| **M14** | Methanolgestarteter EO/PO-Polyether (EO:PO 3:1), Mn 1400 | 1400 |
| **M15** | MPEG 500-gestarteter ε-Caprolactonpolyester Mn 900 | 900 |
| **M16** | iso-Decanolgestarteter ε-Caprolactonpolyester Mn 700 | 700 |
| **M17** | iso-Decanolgestarteter ε-Caprolactonpolyester Mn 1000 | 1000 |
| **M18** | Monophenylglykolgestarteter ε-Caprolactonpolyester, Mn 1200 | 1200 |
| **M19** | n-Butanolgestarteter ε-Caprolactonpolyester, Mn 600 | 600 |
| **M20** | n-Butanolgestarteter ε-Caprolactonpolyester, Mn 1200 | 1200 |
| **M21** | n-Butanolgestarteter PO-Polyether, Mn 1100 | 1100 |
| **M22** | iso-Decanolgestarteter Polyester aus ε-Caprolacton und δ-Valerolacton im molaren Verhältnis 3:1, Mn = 2000 | 2000 |
| **M23** | ε-Caprolactonpolyester Mn 1950, gestartet mit einem n-Butanolgestarteten EO/PO-Polyether (EO:PO 1:1), Mn 1500 | 1950 |
| **M24** | MPEG 350-gestarteter ε-Caprolactonpolyester Mn 900 | 900 |
| **M25** | MPEG 350-gestarteter Polyester aus ε-Caprolacton und δ-Valerolacton im molaren Verhältnis 3:1, Mn = 950 | 950 |
| **M26** | MPEG 500-gestarteter Polyester aus ε-Caprolacton und δ-Valerolacton im molaren Verhältnis 3:1, Mn = 1100 | 1100 |
| **M27** | MPEG 750-gestarteter Polyester aus ε-Caprolacton und δ-Valerolacton im molaren Verhältnis 3:1, Mn = 1400 | 1400 |
| **M28** | MPEG 750 | 750 |
| **M29** | ε-Caprolactonpolyester Mn 1630, gestartet mit einem methanolgestarteten EO/PO-Polyether (EO:PO 3:1), Mn 1400 | 1630 |
| **M30** | n-Butanolgestarteter EO/PO-Polyether (EO:PO 1:1) Mn 2540 | 2540 |
| **M31** | n-Butanolgestarteter PO-Polyether, Mn 2240 | 2240 |
| **M32** | n-Butanolgestarteter Butylenoxid-Polyether, Mn 960 | 960 |
| **M33** | Oleylalkohol | 268 |
| **M34** | Monophenylglykol | 138 |
| **M35** | n-Decanol | 158 |

### Allgemeine Vorschrift zur Herstellung der Amidoamine AA der allgemeinen Formel (IV):

### Erster Schritt (Michael Addition) zu Intermediat I (Tabelle 2):

In einem mit Rührer, Thermometer, Wasserabscheider und Stickstoffeinleitungsrohr versehenen Vierhalskolben werden y g des Amins **B** und/oder z g des Aminoalkohols **C** bei Raumtemperatur vorgelegt. Anschließend werden x g der Komponente **A** langsam zudosiert, so dass die Reaktionstemperatur 35 - 40 °C nicht übersteigt. Der Fortschritt der Reaktion wird mittels 1 H-NMR verfolgt. Die Reaktion gilt als beendet, wenn die C=C-Doppelbindug der ethylenisch ungesättigten Esterverbindung **A** vollständig umgesetzt ist.

### Zweiter Schritt (Amidierung) zu Amidoamin AA (Tabelle 3):

Nach vollständiger Umsetzung der C=C-Doppelbindug der ethylenisch ungesättigten Esterverbindung **A** im ersten Schritt wird die Estergruppe des erhaltenen Intermediats **I** mit einer primären oder sekundären Aminogruppe einer Aminkomponente **B** und/oder **C** amidiert, wobei das Michaeladdukt auf 80 °C erwärmt wird und das Amin **B** und/oder **C** dem Reaktionsgemisch bei 80 °C zugegeben wird und die Temperatur anschließend stufenweise auf 120 °C erhöht wird. Zur besseren Entfernung des entstehenden Alkohols aus dem Reaktionsgemisch wird nach einer Stunde Reaktionszeit bei 120 °C ein Unterdruck von 500 mbar angelegt. Unter diesen Bedingungen wird das Reaktionsgemisch gerührt, bis die Reaktionskontrolle per Infrarotspektroskopie einen vollständigen Umsatz der Estergruppen zu Amidgruppen zeigt. Gegebenenfalls kann nach der Reaktion mit PMA verdünnt werden.

### Herstellung der Intermediate I:

**Tabelle 2: Herstellung der Intermediate I**

| Beispiel | Menge x an Komponente **A** | Menge y an Komponente **B** | Menge z an Komponente **C** |
|---|---|---|---|
| **I1** | 86 g MA | | 105 g DEA |
| **I2** | 172 g MA | 73 g BA | |
| **I3** | 172 g MA | 107 g BzA | |
| **I4** | 86 g MA | 187 g BDMAPA | |
| **I5** | 172 g MA | | 105 g AEE |
| **I6** | 172 g MA | | 61 g EA |
| **I7** | 172 g MA | | 162 g APDEA |
| **I8** | 172 g MA | 102 g DMAPA | |

| | | | |
|---|---|---|---|
| DEA = N,N-Diethanolamin, MA = Methylacrylat, BA = Butylamin, BzA = Benzylamin, BDMAPA = N,N-Bis[3-(dimethylamino)propyl]amin, AEE = 2-(2-Aminoethoxy)ethanol, EA = Ethanolamin, APDEA = N-(3-Aminopropyl)diethanolamin, DMAPA = N,N Dimethylaminopropylamin | | | |

### Herstellung der Amidoamine AA:

**Tabelle 3: Herstellung der Amidoamine AA**

| Beispiel | Menge x an I | Menge y an **B** | Menge z an **C** |
|---|---|---|---|
| **AA1** | 197 g **I1** | 102 g DMAPA | |
| **AA2** | 197 g **I1** | 125 g IPA | |
| **AA3** | 223 g **I6** | 204 g DMAPA | |
| **AA4** | 236 g **I2** | | 122 g EA |
| **AA5** | 236 g **I2** | | 210 g DEA |
| **AA6** | 265 g **I3** | | 122 g EA |
| **AA7** | 265 g **I4** | | 105 g DEA |
| **AA8** | 320 g **I7** | 204 g DMAPA | |
| **AA9** | 263 g **I5** | 204 g DMAPA | |
| **AA10** | 197 g **I1** | 187 g BDMAPA | |
| **AA11** | 266 g **I8** | | 122 g EA |
| **AA12** | 266 g **I8** | | 324 g APDEA |
| **AA13** | 223 g **I6** | | 324 g APDEA |
| **AA14** | 320 g **I7** | | 324 g APDEA |
| **AA15** | 223 g **I6** | 102 g DMAPA | 61 g EA |
| **AA16** | 223 g **I6** | 102 g DMAPA | 162 g APDEA |

| | | | |
|---|---|---|---|
| DMAPA = N,N Dimethylaminopropylamin, IPA = 3-Imidazolyl-1-propylamin, EA = Ethanolamin, DEA = N,N-Diethanolamin, BDMAPA = N,N-Bis[3-(dimethylamino)propyl]amin, APDEA = N-(3-Aminopropyl)diethanolamin | | | |

### Allgemeine Vorschrift für Schritt ii) zur Umsetzung der Monoaddukte M der allgemeinen Formel (III) zu amidoamingruppenhaltigen Reaktionsprodukten der allgemeinen Formel (I):

### In einer einzelnen Stufe ii-a) (Tabelle 4):

In einem mit Rührer, Thermometer, Tropftrichter, Rückflusskühler und Stickstoffeinleitungsrohr versehenen Vierhalskolben werden y g des Amidoamins **AA** vorgelegt und unter Rühren und Stickstoffüberleitung auf 70°C erwärmt. Anschließend werden x g Monoaddukt langsam zudosiert, so dass die Reaktionstemperatur 80 °C nicht übersteigt. Anschließend wird die Reaktionsmischung bei 80 °C gerührt bis das Isocyanat abreagiert ist. Der Fortschritt der Reaktion wird mittels titrimetrischer Bestimmung der NCO-Zahl nach EN ISO 9369 verfolgt. Die Reaktion gilt als beendet, wenn ein NCO-Gehalt von < 0,1% ermittelt wird. Gegebenenfalls kann während oder nach der Reaktion mit PMA verdünnt werden.

Wenn im Amidoamin **AA** gegebenenfalls vorhandene Hydroxylgruppen mit einem Monoaddukt **M** der allgemeinen Formel (III) umgesetzt werden sollen, erfolgt die Umsetzung unter Zugabe von 200 ppm Dibutylzinndilaurat, ansonsten wird die Herstellung wie beschrieben durchgeführt.

**Tabelle 4: Umsetzung der Monoaddukte M mit Amidoaminen AA nach Schritt ii-a)**

| Beispiel | Menge x an Monoaddukt **M** | Menge y an Amidoamin **AA** | PMA in g | Festkörper in % |
|---|---|---|---|---|
| **P1** | 127,4 g **M21** | 26,1 g **AA1** | | 97,8 |
| **P2** | 197,4 g **M10** | 26,1 g **AA1** | | 98,2 |
| **P3** | 442,4 g **M12** | 26,1 g **AA1** | 468,5 g | 49,5 |
| **P4** | 334,8 g **M9** | 26,1 g **AA1** | | 98,6 |
| **P5** | 167,4 g **M9** | 38,6 g **AA3** | | 98,4 |
| **P6** | 97,4 g **M1** | 38,6 g **AA3** | | 97,9 |
| **P7** | 241,4 g **M3** | 38,6 g **AA3** | | 99,2 |
| **P8** | 67,4 g **M7** | 38,6 g **AA3** | | 97,3 |
| **P9** | 157,4 g **M14** | 38,6 g **AA3** | | 98,5 |
| **P10** | 107,4 g **M15** | 38,6 g **AA3** | | 98,7 |
| **P11** | 334,8 g **M9** | 27,6 g **AA2** | | 97,6 |
| **P12** | 167,4 g **M9** | 27,6 g **AA2** | | 97,9 |
| **P13** | 190,8 g **M4** | 27,6 g **AA2** | | 99,1 |
| **P14** | 254,8 g **M8** | 27,6 g **AA2** | | 98,9 |
| **P15** | 154,8 g **M19** | 27,6 g **AA2** | | 97,7 |
| **P16** | 542,8 g **M30** | 27,6 g **AA2** | | 98,5 |
| **P17** | 669,6 g **M9** | 34,8 g **AA5** | | 98,1 |
| **P18** | 309,6 g **M5** | 34,8 g **AA5** | | 99,0 |
| **P19** | 549,6 g **M18** | 34,8 g **AA5** | | 98,8 |
| **P20** | 412,2 g **M18** | 34,8 g **AA5** | | 98,3 |
| **P21** | 274,8 g **M18** | 34,8 g **AA5** | | 97,6 |
| **P22** | 137,4 g **M18** | 34,8 g **AA5** | | 97,1 |
| **P23** | 629,6 g **M27** | 34,8 g **AA5** | 664,4 g | 48,7 |
| **P24** | 124,8 g **M34** | 34,8 g **AA5** | | 97,8 |
| **P25** | 334,8 g **M9** | 30,6 g **AA6** | | 98,4 |
| **P26** | 274,8 g **M6** | 30,6 g **AA6** | | 98,8 |
| **P27** | 104,8 g **M2** | 30,6 g **AA6** | | 98,0 |
| **P28** | 226,8 g **M32** | 30,6 g **AA6** | | 98,1 |
| **P29** | 88,4 g **M33** | 30,6 g **AA6** | | 97,9 |
| **P30** | | 30,6 g **AA6** | | 97,4 |
| **P31** | 334,8 g **M9** | 38,0 g **AA10** | | 96,8 |
| **P32** | 334,8 g **M9** | 31,3 g **AA4** | | 97,5 |
| **P33** | 654,8 g **M11** | 31,3 g **AA4** | | 97,7 |
| **P34** | 482,8 g **M31** | 31,3 g **AA4** | | 97,8 |
| **P35** | 62,4 g **M34** | 31,3 g **AA4** | | 98,1 |
| **P36** | 214,8 g **M24** | 31,3 g **AA4** | | 98,9 |
| **P37** | 334,8 g **M9** | 35,0 g **AA7** | | 98,2 |
| **P38** | 334,8 g **M9** | 44,8 g **AA8** | | 96,4 |
| **P39** | 184,8 g **M28** | 44,8 g **AA8** | | 97,1 |
| **P40** | 92,4 g **M28** | 44,8 g **AA8** | | 96,8 |
| **P41** | 66,4 g **M35** | 44,8 g **AA8** | | 98,8 |
| **P42** | 167,4 g **M9** | 38,8 g **AA9** | | 99,2 |
| **P43** | 117,4 g **M17** | 38,8 g **AA9** | | 97,4 |
| **P44** | 92,4 g **M29** | 38,8 g **AA9** | | 98,2 |
| **P45** | 334,8 g **M9** | 30,2 g **AA11** | | 98,8 |
| **P46** | 274,8 g **M20** | 30,2 g **AA11** | | 96,7 |
| **P47** | 224,8 g **M25** | 30,2 g **AA11** | 255,0 g | 49,8 |
| **P48** | 669,6 g **M9** | 47,1 g **AA12** | | 97,6 |
| **P49** | 502,2 g **M9** | 47,1 g **AA12** | | 98,6 |
| **P50** | 334,8 g **M9** | 47,1 g **AA12** | | 98,2 |
| **P51** | 167,4 g **M9** | 47,1 g **AA12** | | 98,7 |
| **P52** | 509,6 g **M26** | 47,1 g **AA12** | | 97,8 |
| **P53** | 509,6 g **M21** | 47,1 g **AA12** | | 99,0 |
| **P54** | 669,6 g **M9** | 50,8 g **AA13** | | 99,4 |
| **P55** | 349,6 g **M16** | 50,8 g **AA13** | | 98,8 |
| **P56** | 849,6 g **M23** | 50,8 g **AA13** | | 98,2 |
| **P57** | 502,2 g **M9** | 53,6 g **AA14** | | 99,1 |
| **P58*** | 167,4 g **M9** | 10,2 g **DMAPA** | | 98,4 |
| **P59*** | 167,4 g **M9** | 8,9 g **DMAE** | | 96,5 |
| **P60*** | 167,4 g **M9** | 18,7 g **BDMAPA** | | 97,6 |
| **P61*** | 167,4 g **M9** | 14,9 g **TEA** | | 98,0 |
| **P62** | 274,8 g **M13** | 32,2 g **AA15** | | 97,8 |
| **P63** | 652,2 g **M22** | 41,7 g **AA16** | | 98,4 |

| | | | | |
|---|---|---|---|---|
| * nicht erfindungsgemäße Beispiele/Stand der Technik PMA = Methoxypropylacetat (Lösemittel), DMAPA = N,N-Dimethylaminopropylamin, TEA = Triethanolamin, DMAE = N,N-Dimethylaminoethanol, BDMAPA = N,N-Bis[3-(dimethylamino)propyl]amin | | | | |

### In einer Stufenabfolge ii-b) (Tabelle 5):

### Erster Schritt:

In einem mit Rührer, Thermometer, Tropftrichter, Rückflusskühler und Stickstoffeinleitungsrohr versehenen Vierhalskolben werden y g des Intermediats **I** vorgelegt und unter Rühren und Stickstoffüberleitung auf 70 °C erwärmt. Anschließend werden x g Monoaddukt **M** langsam zudosiert, so dass die Reaktionstemperatur 80 °C nicht übersteigt. Anschließend wird die Reaktionsmischung bei 80 °C gerührt bis das Isocyanat abreagiert ist. Der Fortschritt der Reaktion wird mittels titrimetrischer Bestimmung der NCO-Zahl nach EN ISO 9369 verfolgt. Die Reaktion gilt als beendet, wenn ein NCO-Gehalt von < 0,1% ermittelt wird.

### Zweiter Schritt:

Nach vollständiger Umsetzung der Isocyanatgruppe mit einer Alkoholgruppe, einer primären Aminogruppe oder einer sekundären Aminogruppe eines Intermediats **I** im ersten Schritt wird das erhaltenen Zwischenprodukt **J** mit einer primären oder sekundären Aminogruppe einer Aminkomponente **B** und/oder **C** amidiert, wobei das Amin **B** und/oder **C** dem Reaktionsgemisch bei 80 °C zugegeben wird und die Temperatur stufenweise auf 120 °C erhöht wird. Zur besseren Entfernung des entstehenden Alkohols aus dem Reaktionsgemisch wird nach einer Stunde Reaktionszeit bei 120 °C ein Unterdruck von 500 mbar angelegt. Unter diesen Bedingungen wird das Reaktionsgemisch gerührt, bis die Reaktionskontrolle per Infrarotspektroskopie einen vollständigen Umsatz der Estergruppe zur Amidgruppe zeigt. Bei Produkten, die Polyester im Y-Rest enthalten, so dass die Reaktionskontrolle per Infrarotspektroskopie erschwert wird, wird das Ende der Reaktion dadurch festgelegt, dass sich kein weiterer Alkohol bildet, somit gilt die Amidierung als beendet. Gegebenenfalls kann nach der Reaktion mit PMA verdünnt werden.

**Tabelle 5: Umsetzung der Monoaddukte M mit einem Intermediat I und anschließender Amidierung nach Schritt ii-b)**

| Beispiel | Menge x an Monoaddukt **M** | Menge y an Intermediat **I** | Menge z an Komponente **B** oder **C** |
|---|---|---|---|
| **P64** | 127,4 g **M21** | 19,7 g **I1** | 10,2 g **DMAPA** |
| **P65** | 254,8 g **M21** | 19,7 g **I1** | 10,2 g **DMAPA** |
| **P66** | 97,4 g **M1** | 22,3 g **I6** | 20,4 g **DMAPA** |
| **P67** | 194,8 g **M1** | 19,7 g **I1** | 10,2 g **DMAPA** |
| **P68** | 127,4 g **M21** | 19,7 g **I1** | 6,1 g **EA** |

| | | | |
|---|---|---|---|
| DMAPA = N,N Dimethylaminopropylamin, EA = Ethanolamin | | | |

### Allgemeine Vorschrift zur Quaternisierung (Tabelle 6):

In einem mit Rührer, Thermometer, Tropftrichter, Rückflusskühler und Stickstoffeinleitungsrohr versehenen Vierhalskolben werden y g der Ausgangsverbindung in 170 g PMA (Methoxypropylacetat) und 170 g Butylglykol sowie x g Alkylierungsreagenz 4 h bei 120 °C zur Reaktion gebracht. Der Festkörper wird mit einer 1:1-Mischung von PMA und Butylglykol auf 40% eingestellt.

**Tabelle 6: Quarternisierung**

| Beispiel | Menge y an Ausgangsverbindung | Menge x an Alkylierungsreagenz |
|---|---|---|
| **Q1** | 224 g **P2** | 6,9 g Benzylchlorid |
| **Q2** | 224 g **P2** | 14,7 g Grilonit 1814 6,4 g Benzoesäure |
| **Q3** | 224 g **P2** | 9,5 g Benzylchlorid |
| **Q4** | 224 g **P2** | 26,7 g Grilonit 1814 11,6 g Benzoesäure |
| **Q5** | 224 g **P2** | 12,0 g Benzylchlorid |

| | | |
|---|---|---|
| Grilonit RV 1814 = C₁₃/C₁₅-Alkylglycidylether, EMS-Chemie | | |

### Allgemeine Vorschrift zur Salzbildung (Tabelle 7):

In einem mit Rührer, Thermometer, Tropftrichter, Rückflusskühler und Stickstoffeinleitungsrohr versehenen Vierhalskolben werden y g der Ausgangsverbindung in 170 g PMA und 170 g Butylglycol mit x g Versalzungsreagenz 1 h bei 60 °C gerührt.

**Tabelle 7: Salzbildung**

| **Beispiel** | **Menge y an Reaktionsprodukt P** | **Menge x an Versalzungsreagenz** |
|---|---|---|
| **S1** | 224 g **P2** | 14,6 g Adipinsäure |
| **S2** | 224 g **P2** | 24,4 g Benzoesäure |
| **S3** | 224 g **P2** | 12,2 g Benzoesäure |
| **S4** | 224 g **P2** | 7,3 g Adipinsäure |
| **S5** | 224 g **P2** | 28,2 g Ölsäure |
| **S6** | 224 g **P2** | 20,0 g Laurinsäure |
| **S7** | 224 g **P2** | 29,8 g Ricinolsäure |
| **S8** | 224 g **P2** | 9,0 g Milchsäure |

### c) Anwendungstechnische Abprüfung

Verwendung der erfindungsgemäßen Polymere als Netz- und Dispergieradditiv zur Herstellung von Pigmentkonzentraten und deren Einsatz in Lacksystemen.

**Ausgangsstoffe**

| | |
|---|---|
| Paraloid B-66 | thermoplastisches Acrylatharz, Hersteller DOW Chemicals |
| Joncryl 500 | Hydroxyfunktionelles Acryl-Harz, Hersteller BASF |
| Laropal A 81 | Aldehyd-Harz, Hersteller BASF |
| Cymel 303 | Amino-Crosslinker, Hersteller Allnex |
| MAK | Methylamylketon, Hersteller Eastman |
| DIDP | Diisodecylphthalat |
| Nacure 2500 | p-Toluolsulfonsäure, Hersteller King Industries |
| Dowanol PMA | Propylenglykolmethyletheracetat, Hersteller Dow Chemicals |
| Raven 5000 Ultra III | Rußpigment für hohe Jetness, Hersteller |
| Paliogen Red L3880 HD | Perylen-Rot Pigment, Hersteller |
| Hostaperm RL NF | Violett-Pigment, Hersteller |
| Heliogen Blue L7101 F | Phthalocyanin-Blau (P.B. 15:4), Hersteller BASF |
| Hostaperm Rosa E | Chinacridon-Rot (P.R. 122), Hersteller Clariant |
| Colour Black FW 200 | Rußpigment (P. Bk. 7), Hersteller Orion |
| BYK 310 | Substratnetzmittel, Hersteller BYK-Chemie |
| BYK 306 | Substratnetzmittel, Hersteller BYK-Chemie |

### Arbeitsverfahren

### Herstellung von Pigmentkonzentraten auf Basis eines thermoplastischen Acrylats

Das Anreibeharz Paraloid B-66, Lösemittel, Dispergieradditiv und Pigment wurden in 100mL Glasflaschen so eingewogen, dass 50g Mahlgut erhalten wurden. Anschließend wurden 50g Glasperlen (1 mm) eingewogen.

**Zusammensetzung der TPA-Pigmentkonzentrate (Angaben in g)**

| | **TPA 1 (schwarz)** | **TPA 2 (rot)** | **TPA 3 (violett)** |
|---|---|---|---|
| Paraloid B-66 (50% in Xylol) | 24,0 | 24,0 | 24,0 |
| Raven Ultra 5000 III | 6,0 | | |
| Paliogen Red L 3880 HD | | 8,0 | |
| Hostaperm RL-NF | | | 5,0 |
| Dispergieradditiv | 4,2 | 2,0 | 2,0 |
| n-Butanol | 5,0 | 5,0 | 5,0 |
| PMA | | 11,0 | 14,0 |
| Butylacetat | 10,8 | | |
| Gesamtpigmentgehalt (%) | 12 | 16 | 10 |
| Dispergiermittel (% s.o.p.) | 70 | 25 | 40 |

### Mahlbedingungen:

Gerät: Lau Disperser DAS 200
Dispergierzeit: 300 min, Kühlleistung Stufe 3
Verhältnis Mahlgut zu Glasperlen (1 mm Durchmesser): 1 : 1 (Gewichtsteile)

### Bewertung der Mahlgutviskosität der TPA-Anreibungen

Die Mahlgutviskosität der TPA-Anreibungen wurde ermittelt mit einem Rheological Stresstech Rheometer (Platte/Kegel, 25mm, 1 °) bei 23°C.

**Mahlgutviskositäten**

| | Viskosität in Pa * s | | | | | | | | |
|---|---|---|---|---|---|---|---|---|---|
| | 1/s | 10/s | 100/s | 1/s | 10/s | 100/s | 1/s | 10/s | 100/s |
| *P59* | 45,890 | 7,727 | 1,828 | 15,370 | 2,876 | 0,814 | 42,110 | 4,786 | 1,085 |
| P60* | 48,380 | 7,022 | 1,663 | 15,440 | 2,879 | 0,864 | 38,460 | 4,680 | 1,005 |
| P61* | 51,610 | 7,534 | 1,774 | 22,470 | 3,808 | 1,011 | 57,960 | 7,762 | 1,432 |
| P4 | 12,200 | 2,688 | 1,048 | 7,456 | 1,722 | 0,657 | 35,530 | 4,294 | 0,970 |
| P11 | 24,470 | 4,746 | 1,419 | 9,462 | 1,881 | 0,677 | 37,990 | 4,614 | 0,968 |
| P17 | 32,960 | 6,113 | 1,631 | 6,069 | 1,586 | 0,685 | 36,670 | 4,508 | 0,982 |
| P25 | 18,390 | 3,661 | 1,132 | 8,977 | 1,888 | 0,688 | 39,180 | 4,647 | 1,027 |

Die erfindungsgemäßen Dispergieradditive P4, P11, P17 und P25 wirken stark viskositätsreduzierend im Vergleich zum Stand der Technik P59*, P60* und P61* bei TPA-Anreibungen von 3 verschieden Pigmenten, was sich schon bei niedrigen Scherraten bemerkbar macht.

### Herstellung des TPA-basierten Auflacksystems

Paraloid B-66, Lösemittel und Verlaufsadditiv wurden in einem 2,5L PE-Eimer eingewogen und mit einem Dispermat CV (Zahnscheibe 65mm) bei 2000rpm 5min homogenisiert.

**Zusammensetzung des TPA-Auflacksystems (Angaben in g)**

| TPA-Klarlack | Einwaage in g |
|---|---|
| Paraloid B-66 (50% in Xylol) | 700 |
| DIDP | 20 |
| Xylol | 218 |
| PMA | 60 |
| BYK-306 | 2 |

### Herstellung der pigmentierten TPA-Auflacksysteme

In einem PE-Becher wurden das TPA-Auflacksystem und die TPA-basierte Pigmentanreibung eingewogen und mit einem Spatel vermischt. Anschließend wurden alle finalen TPA-Auflacksysteme für 10min in einem ANDALOK-Shaker homogenisiert.

**Zusammensetzung der pigmentierten TPA-Auflacksysteme (Angaben in g)**

| | TPA-B1 | TPA-B2 | TPA-B3 |
|---|---|---|---|
| TPA-Auflacksystem | 28.0 | 27,0 | 26,0 |
| TPA 1 (schwarz) | 2,0 | | |
| TPA 2 (rot) | | 3,0 | |
| TPA 3 (violett) | | | 4,0 |
| Pigmentgehalt (%) | 0,8 | 1,6 | 1,3 |

### Applikation und Auswertung der pigmentierten TPA-Auflacksvsteme

Die pigmentierten TPA-Auflacksysteme wurden auf PE-Folie aufgerakelt (50 µm bzw. 100 µm) und 24h bei 22°C getrocknet. Anschließend wurden der Glanzschleier und der Glanz mit einem BYK micro haze plus Instrument im Winkel von 20°gemessen. Dabei sind jeweils niedrige Werte für den Glanzschleier und hohe Werte für den Glanz als positive Ergebnisse zu bewerten. Außerdem wurde die optische Farbstärke und Transparenz der Aufzüge auf PE-Folie in der Durchsicht mit den Noten 1 (hervorragend) bis 5 (nicht akzeptabel) bewertet.

| **TPA B1 (schwarz)** Raven 5000 Ultra III | | | | | |
|---|---|---|---|---|---|
| **Dispergieradditiv im Mahlgut** | **Vollton** 100µm PE-Folie **Glanzschleier** | | **Vollton** 100µm PE-Folie **Glanz 20°** | | **Vollton** 100µm PE-Folie **Transparenz/Farbstärke** |
| P59* | 11 | | 80 | | 2-3 |
| P60* | 21 | | 78 | | 4 |
| P61* | 12 | | 80 | | 3 |
| P4 | 11 | | 80 | | 2-3 |
| P11 | 10 | | 81 | | 2 |
| P17 | 10 | | 82 | | 1-2 |
| P25 | 11 | | 80 | | 2 |

| **TPA B2 (rot)** Paliogen Red L3880 HD | | | | | |
|---|---|---|---|---|---|
| **Dispergieradditiv im Mahlgut** | | **Vollton** 100µm PE-Folie **Glanzschleier** | | **Vollton** 100µm PE-Folie **Glanz 20°** | **Vollton** 50µm PE-Folie **Transparenz/Farbstärke** |
| P59* | | 13 | | 77 | 3-4 |
| P61* | | 20 | | 73 | 4 |
| P4 | | 11 | | 80 | 3 |
| P17 | | 12 | | 79 | 3 |

| TPA B3 (violett) Hostaperm RL-NF | | | | | |
|---|---|---|---|---|---|
| **Dispergieradditiv im Mahlgut** | **Vollton** 50µm PE-Folie **Glanzschleier** | | **Vollton** 50µm PE-Folie **Glanz 20°** | | **Vollton** 50µm PE-Folie **Transparenz/Farbstärke** |
| P59* | 43 | | 71 | | 3-4 |
| P61* | 45 | | 71 | | 3 |
| P4 | 36 | | 74 | | 2-3 |
| P25 | 36 | | 75 | | 2-3 |

Die erfindungsgemäßen Dispergieradditive zeigen einen geringeren Glanzschleier, bessere Glanzwerte und eine höhere Transparenz sowie Farbstärke im Vergleich zum Stand der Technik für TPA-basierte Lacksysteme.

### Herstellung der Laropal A81-Pigmentanreibung

Das Anreibeharz Laropal A81 (60 Teile) wurde mit PMA (40 Teile) in einem 2,5L PE-Eimer eingewogen und mittels eines Dispermaten CV (65mm Zahnscheibe) bei 2000 rpm für 30min homogenisiert. Anschließend wurden die Lösung des Anreibeharzes, Lösemittel, Dispergieradditiv und Pigment in 100mL Glasflaschen so eingewogen, dass 50g Mahlgut erhalten wurden. Anschließend wurden 50g Glasperlen (1mm) eingewogen.

**Zusammensetzung der Laropal-A81-Pigmentkonzentrate (Angaben in g)**

| | LA 1 (schwarz) | LA 2 (rosa) | LA 3 (blau) |
|---|---|---|---|
| Laropal A 81 (60%ig in PMA) | 8,7 | 20,4 | 21,9 |
| Colour Black FW 200 | 4,0 | | |
| Hostaperm Rosa E | | 7,0 | |
| Heliogen Blau 7101 F | | | 7,5 |
| Dispergieradditiv | 2,8 | 1,8 | 1,9 |
| PMA | 34,5 | 20,8 | 18,7 |
| Gesamtpigmentgehalt (%) | 8 | 14 | 15 |
| Dispergiermittel (% s.o.p.) | 70 | 25 | 25 |

### Mahlbedingungen:

Gerät: Lau Disperser DAS 200
Mahlzeit: 300 min, Kühlleistung Stufe 3
Verhältnis Mahlgut zu Glasperlen (1 mm Durchmesser): 1 : 1 (Gewichtsteile)

### Bewertung der Mahlgutviskosität und der Laropal-A81-Anreibungen

Die Mahlgutviskosität der Laropal A81-Anreibungen wurde ermittelt mit einem Rheological Stresstech Rheometer (Platte/Kegel, 25mm, 1 °) bei 23°C.

**Mahlgutviskositäten**

| | Viskosität in mPa * s | | | Viskosität in mPa * s | | | Viskosität in mPa * s | | |
|---|---|---|---|---|---|---|---|---|---|
| | 1/s | 10/s | 100/s | 1/s | 10/s | 100/s | 1/s | 10/s | 100/s |
| **P58*** | 44 | 13 | 10 | 34870 | 3934 | 794 | 9457 | 1017 | 214 |
| **P60*** | 185 | 86 | 34 | 28460 | 3656 | 939 | 10450 | 1474 | 228 |
| **P61*** | 232 | 25 | 9 | 33660 | 3399 | 995 | 10720 | 815 | 247 |
| **P4** | 26 | 12 | 9 | 25750 | 2907 | 519 | 6646 | 894 | 179 |
| **P11** | 36 | 12 | 9 | 24990 | 3252 | 519 | 6072 | 827 | 172 |
| **P17** | 19 | 11 | 9 | 18400 | 1857 | 381 | 7889 | 905 | 202 |
| **P25** | 31 | 13 | 9 | 25230 | 2841 | 555 | 6862 | 921 | 184 |

### Herstellung eines Einbrennlacks JC1

Bindemittel, Lösemittel und Substratnetzmittel wurden in einem 2,5L PE-Eimer eingewogen und mit einem Dispermat CV (Zahnscheibe 65mm) bei 2000rpm 5min homogenisiert.

**Zusammensetzung des Einbrennlacks JC1 (Angaben in g)**

| **Einbrennlack JC1** | |
|---|---|
| Joncryl 500 | 576,0 |
| Cymel 303 | 198,0 |
| Butanol | 80,0 |
| MAK | 130,0 |
| BYK-310 | 3,0 |
| Nacure 2500 | 13,0 |

### Herstellung verschiedener Lacke auf Basis der Laropal A81-Pigmentanreibungen

In einem PE-Becher wurden die Laropal A81-basierten Pigmentanreibungen und der Einbrennlack JC1 eingewogen und per Hand mit einem Spatel vermischt. Anschließend wurden die pigmentierten Lacke für 10min in einem ANDALOK-Shaker homogenisiert.

### Zusammensetzung der pigmentierten JC1-Auflacksysteme (Angaben in g)

Applikation und Auswertung der pigmentierten Einbrennlacke JC1-B1 bis JC1-B3

Die pigmentierten Einbrennlacke JC1-B1 bis JC1-B3 wurden auf PE-Folie aufgerakelt (50 µm), 15min bei 22°C abgelüftet und 20min bei 150°C eingebrannt. Anschließend wurden Glanzschleier und Glanz mit einem BYK micro haze plus Instrument im Winkel von 20° gemessen. Dabei sind jeweils niedrige Werte für den Glanzschleier und hohe Werte für den Glanz als positive Ergebnisse zu bewerten. Außerdem wurde die optische Transparenz und die Farbstärke der Aufzüge auf PE-Folie in der Durchsicht mit den Noten 1 (hervorragend) bis 5 (nicht akzeptabel) bewertet.

**Vergleich der schwarzen Einbrennlacke JC1-B1 (schwarz)**

| **Synthese-Name** | **Glanz 20°** | **Glanzschleier** | **Transparenz / Farbstärke** |
|---|---|---|---|
| **P58*** | 106 | 11 | 4 |
| **P60*** | 103 | 24 | 3-4 |
| **P61*** | 105 | 14 | 1-2 |
| **P4** | 106 | 14 | 1 |
| **P11** | 105 | 10 | 1 |
| **P17** | 106 | 16 | 1 |
| **P25** | 107 | 10 | 2 |

**Vergleich der rosa-farbenen Einbrennlacke JC1-B2 (rosa)**

| **Synthese-Name** | **Glanz 20°** | **Glanzschleier** | **Transparenz / Farbstärke** |
|---|---|---|---|
| **P59*** | 112 | 33 | 3 |
| **P60*** | 113 | 19 | 4 |
| **P61*** | 113 | 21 | 4 |
| **P4** | 116 | 14 | 3-4 |
| **P11** | 115 | 20 | 3 |
| **P17** | 116 | 18 | 3 |
| **P25** | 115 | 14 | 3-4 |

**Vergleich der blauen Einbrennlacke JC1-B3 (blau)**

| **Synthese-Name** | **Glanz 20°** | **Glanzschleier** | **Transparenz / Farbstärke** |
|---|---|---|---|
| **P59*** | 115 | 21 | 4 |
| **P60*** | 112 | 38 | 3 |
| **P61*** | 114 | 27 | 3-4 |
| **P4** | 115 | 26 | 2 |
| **P11** | 116 | 27 | 1 |
| **P17** | 115 | 24 | 3 |
| **P25** | 117 | 20 | 2 |

Die erfindungsgemäßen Dispergieradditive P4, P11, P17 und P25 zeigen einen geringeren Glanzschleier, bessere Glanzwerte und eine höhere Transparenz sowie Farbstärke im Vergleich zum Stand der Technik P59*,P60* und P61* für Pigmentkonzentrate auf Basis von Laropal A81 eingesetzt in einem säurekatalysierten Einbrennlack.

## Patentansprüche

1. Amidoamingruppenhaltiges Reaktionsprodukt, enthaltend ein oder mehrere Spezies gemäß der allgemeinen Formel (I)
(R¹-X)ₚ-Z¹⁻(XH)_{y} (I),
wobei gilt p + y = w und
w für eine ganze Zahl von 1 bis 10 steht,
p für eine ganze Zahl von 1 bis 10 steht,
y für eine ganze Zahl von 0 bis 9 steht, und
X unabhängig voneinander für O, NH und/oder NZ² steht und XH unabhängig voneinander für eine Hydroxylgruppe OH, eine primäre Aminogruppe NH₂ und/oder eine sekundäre Aminogruppe NHZ² steht, wobei
Z² unabhängig voneinander für einen verzweigten oder unverzweigten, gesättigten oder ungesättigten organischen Rest G(U)ₐ steht, wobei U unabhängig voneinander für eine Hydroxylgruppe, eine primäre Aminogruppe oder eine sekundäre Aminogruppe steht und
a für eine ganze Zahl von 0 bis 9 steht,
wobei gilt p + y + a ≤ 10, und
G für einen verzweigten oder unverzweigten, gesättigten oder ungesättigten organischen Rest steht,
die p Reste R¹ unabhängig voneinander für einen Rest der allgemeinen Formel (II)
Y-O-CO-NH-R ²⁻NH-CO (II)
stehen,
worin die p Reste Y unabhängig voneinander für einen verzweigten oder unverzweigten, gesättigten oder ungesättigten organischen Rest mit 1 bis 1000 Kohlenstoffatomen stehen, der keine Hydroxylgruppen, keine primären Aminogruppen und keine sekundären Aminogruppen enthält,
die p Reste R² unabhängig voneinander für einen verzweigten oder unverzweigten, gesättigten oder ungesättigten organischen Rest mit 6 bis 20 Kohlenstoffatomen stehen,
Z¹ für einen mindestens zwei Kohlenstoffatome enthaltenden verzweigten oder unverzweigten, gesättigten oder ungesättigten organischen Rest steht, der mindestens eine Amidgruppe und mindestens eine tertiäre Aminogruppe aufweist.

2. Amidoamingruppenhaltiges Reaktionsprodukt nach Anspruch 1 enthaltend mindestens 40 Gew.-%, bevorzugt mindestens 60 Gew.-%, besonders bevorzugt mindestens 90 Gew.-% einer oder mehrerer Spezies der allgemeinen Formel (I).

3. Amidoamingruppenhaltiges Reaktionsprodukt nach Anspruch 1 oder 2, wobei die zur Formel (I) korrespondierende, urethangruppenfreie, gegenüber isocyanatgruppen reaktive Spezies gemäß der allgemeinen Formel (IV)
(HX)ₚ-Z¹-(HX)_{y} (IV),
mit p, y, X und Z¹ wie für die allgemeine Formel (I) definiert,
durch Umsetzung einer oder mehrerer Komponenten A mit einer oder mehrerer Komponenten B und/oder C erhalten wird, wobei
die Komponente A gewählt wird aus der Gruppe ethylenisch ungesättigter Carbonsäuren, deren Estern und deren Säurehalogeniden, wobei mindestens eine C=C-Doppelbindung und mindestens eine C=O-Doppelbindung konjugiert vorliegen und die C=O-Doppelbindung gewählt wird aus der Gruppe der Carbonsäuren, der Carbonsäureester und Carbonsäurehalogenide;
die Komponente B gemäß der allgemeinen Formel (V)
(R³)x-HN-(R⁴)_{z} (V)
vorliegt,
wobei gilt x + z = 2 und
x für eine ganze Zahl von 0 bis 2 steht,
z für eine ganze Zahl von 0 bis 2 steht, und
R³ unabhängig voneinander für H oder einen verzweigten oder unverzweigten, gesättigten oder ungesättigten organischen Rest mit 1 bis 12 Kohlenstoffatomen steht, und
R⁴ unabhängig voneinander für einen verzweigten oder unverzweigten, gesättigten oder ungesättigten organischen Rest mit 2 bis 12 Kohlenstoffatomen und 1 bis 3 tertiären Aminogruppen steht; und
die Komponente C gemäß der allgemeinen Formel (VI)
(R⁵)ₖ-HN-(R⁶)ₙ (VI)
vorliegt,
wobei gilt k + n = 2 und
k für eine ganze Zahl von 0 bis 1 steht,
n für eine ganze Zahl von 1 bis 2 steht, und
R⁵ für H oder einen verzweigten oder unverzweigten, gesättigten oder ungesättigten organischen Rest mit 1 bis 12 Kohlenstoffatomen steht, und
R⁶ unabhängig voneinander für einen verzweigten oder unverzweigten, gesättigten oder ungesättigten organischen Rest mit 2 bis 12 Kohlenstoffatomen und 1 bis 4 Hydroxylgruppen steht.

4. Amidoamingruppenhaltiges Reaktionsprodukt nach Anspruch 3, wobei die Komponente A ein ethylenisch ungesättigter Carbonsäureester ist.

5. Amidoamingruppenhaltiges Reaktionsprodukt nach einem der Ansprüche 1 bis 4, wobei das zum Rest R² korrespondierende Diisocyanat R²(NCO)₂ zwei unterschiedlich reaktive Isocyanatgruppen besitzt.

6. Amidoamingruppenhaltiges Reaktionsprodukt nach einem der Ansprüche 1 bis 5, wobei Y mindestens einen Polyetherrest, Polyesterrest, Kohlenwasserstoffrest und/oder Polysiloxanrest enthält.

7. Verfahren zur Herstellung eines amidoamingruppenhaltigen Reaktionsproduktes nach einem der Ansprüche 1 bis 6, wobei
i) mindestens ein Diisocyanat R²(NCO)₂ mit mindestens einem Monoalkohol Y-OH unter Ausbildung eines Urethans der allgemeinen Formel (III)
Y-O-CO-NH-R²-NCO (III)
umgesetzt wird, wobei
R² und Y wie in Ansprüchen 1 bis 6 definiert sind, und
ii) p Urethane der allgemeinen Formel (III) zu einem amidoamingruppenhaltigen Reaktionsprodukt, enthaltend ein oder mehrere Spezies gemäß der allgemeinen Formel (I)
(R¹-X)ₚ-Z¹-(XH)_{y} (I),
umgesetzt werden, wobei y, p, R¹, X und Z¹ wie in Ansprüchen 1 bis 6 definiert sind, und
wobei Schritt ii) in einer Stufe ii-a) oder in einer Stufenabfolge ii-b) durchgeführt wird,
in der Stufe ii-a) p Urethane der allgemeinen Formel (III) mit einer urethangruppenfreien, gegenüber Isocyanatgruppen reaktiven Spezies gemäß der allgemeinen Formel (IV) nach Anspruch 3 umgesetzt werden, und
in der Stufe ii-b) p Urethane der allgemeinen Formel (III) mit einem Intermediat I, welches mindestens eine gegenüber Isocyanatgruppen reaktive Gruppe HX enthält, mit X wie für die allgemeine Formel (I) definiert, umgesetzt werden unter Ausbildung eines Zwischenproduktes J, wobei I ein Michaeladditionsprodukt mindestens einer Komponente A mit einer Komponente B oder C ist, und anschließend das entstandene Zwischenprodukt J in einer Amidierungsreaktion mit einer Komponente B oder C umgesetzt wird.

8. Verfahren nach Anspruch 7, wobei in Schritt i) das Diisocyanat R²(NCO)₂ zum Monoalkohol Y-OH in einem molaren Verhältnis von mindestens 1,1:1,0 eingesetzt wird und das nicht in Schritt i) zum Urethan der allgemeinen Formel (III) umgesetzte Diisocyanat R²(NCO)₂ vor der Durchführung des Schrittes ii) aus dem Reaktionsgemisch, bevorzugt durch Destillation, entfernt wird.

9. Verfahren nach einem der Ansprüche 7 oder 8, wobei das zum Rest R² korrespondierende Diisocyanat R²(NCO)₂ zwei unterschiedlich reaktive Isocyanatgruppen besitzt.

10. Verfahren nach Anspruch 9, wobei das Diisocyanat R²(NCO)₂ ausgewählt wird aus der Gruppe bestehend aus 2,4-Toluoldiisocyanat und Isophorendiisocyanat.

11. Netz- und Dispergiermittel enthaltend ein amidoamingruppenhaltiges Reaktionsprodukt gemäß einem der Ansprüche 1 bis 6 oder hergestellt nach einem Verfahren gemäß der Ansprüche 7 bis 10.

12. Zusammensetzung enthaltend ein amidoamingruppenhaltiges Reaktionsprodukt gemäß einem der Ansprüche 1 bis 6 oder hergestellt nach einem Verfahren gemäß der Ansprüche 7 bis 10.

13. Verwendung eines amidoamingruppenhaltigen Reaktionsprodukts gemäß einem der Ansprüche 1 bis 6 oder hergestellt nach einem Verfahren gemäß der Ansprüche 7 bis 10 als Additiv, bevorzugt als Netzmittel oder Dispergiermittel oder Dispersionsstabilisator oder Viskositätsreduzierer oder Kompatibilisator in Beschichtungen, insbesondere Lacken, Kunststoffen, Pigmentpasten, Dichtstoffen, Kosmetika, Keramik, Klebstoffen, Vergussmassen, Spachtelmassen, Druckfarben und Tinten.

14. Amidoamingruppenhaltiges Reaktionsprodukt, wobei dieses eine Spezies der allgemeinen Formel (I) gemäß Anspruch 1 ist.

15. Amidoamingruppenhaltiges Reaktionsprodukt enthaltend ein oder mehrere Salze und/oder ein oder mehrere Quaternisierungsprodukte der Spezies der allgemeinen Formel (I) nach Anspruch 1.

16. Amidoamingruppenhaltiges Reaktionsprodukt enthaltend ein oder mehrere Modifikationsprodukte der Spezies der allgemeinen Formel (I) nach Anspruch 1, wobei die eine oder die mehreren tertiären Aminogruppen der allgemeinen Formel (I) mit Sauerstoff und/oder Peroxoverbindungen in Aminoxide überführt wurden und/oder noch vorhandene XH-Gruppen in der allgemeinen Formel (I) mit Carbonsäureanhydriden umgesetzt wurden.

## Claims

1. Reaction product containing amido amine groups, comprising one or more species of the general formula (I)
(R¹-X)ₚ-Z¹-(XH)_{y} (I)
where p + y = w and
w is an integer from 1 to 10,
p is an integer from 1 to 10,
y is an integer from 0 to 9, and
X is independently O, NH and/or NZ² and XH is independently a hydroxyl group OH, a primary amino group NH₂ and/or a secondary amino group NHZ² where
Z² is independently a branched or unbranched, saturated or unsaturated organic radical G(U)ₐ where U is independently a hydroxyl group, a primary amino group or a secondary amino group and
a is an integer from 0 to 9,
where p + y + a ≤ 10, and
G is a branched or unbranched, saturated or unsaturated radical,
the p R¹ radicals are independently a radical of the general formula (II)
Y-O-CO-NH-R²-NH-CO (II)
in which the p Y radicals are independently a branched or unbranched, saturated or unsaturated organic radical which has 1 to 1000 carbon atoms and does not contain any hydroxyl groups, any primary amino groups or any secondary amino groups,
the p R² radicals are independently a branched or unbranched, saturated or unsaturated organic radical having 6 to 20 carbon atoms,
Z¹ is a branched or unbranched, saturated or unsaturated
organic radical containing at least two carbon atoms, having at least one amide group and at least one tertiary amino group.

2. Reaction product containing amido amine groups according to Claim 1, containing at least 40% by weight, preferably at least 60% by weight and more preferably at least 90% by weight of one or more species of the general formula (I).

3. Reaction product containing amido amine groups according to Claim 1 or 2, wherein the species of the general formula (IV) which is free of urethane groups, is reactive toward isocyanate groups and corresponds to the formula (I)
(HX)ₚ-Z¹-(HX)_{y} (IV)
with p, y, X and Z¹ as defined for the general formula (I)
is obtained by reacting one or more components A with one or more components B and/or C, wherein
component A is selected from the group of ethylenically unsaturated carboxylic acids, esters thereof and acid halides thereof, where at least one C=C double bond and at least one C=O double bond are in conjugated form and the C=O double bond is selected from the group of the carboxylic acids, the carboxylic esters and the carbonyl halides;
component B is of the general formula (V)
(R³)ₓ-HN-(R⁴)_{z} (V)
where x + z = 2 and
x is an integer from 0 to 2,
z is an integer from 0 to 2, and
R³ is independently H or a branched or unbranched, saturated or unsaturated organic radical having 1 to 12 carbon atoms, and
R⁴ is independently a branched or unbranched, saturated or unsaturated organic radical having 2 to 12 carbon atoms and 1 to 3 tertiary amino groups; and
component C is of the general formula (VI)
(R⁵)ₖ-HN-(R⁶)ₙ (VI)
where k + n = 2 and
k is an integer from 0 to 1,
n is an integer from 1 to 2, and
R⁵ is H or a branched or unbranched, saturated or unsaturated organic radical having 1 to 12 carbon atoms, and
R⁶ is independently a branched or unbranched, saturated or unsaturated organic radical having 2 to 12 carbon atoms and 1 to 4 hydroxyl groups.

4. Reaction product containing amido amine groups according to Claim 3, wherein component A is an ethylenically unsaturated carboxylic ester.

5. Reaction product containing amido amine groups according to any of Claims 1 to 4, wherein the diisocyanate R² (NCO)₂ corresponding to the R² radical has two isocyanate groups of different reactivity.

6. Reaction product containing amido amine groups according to any of Claims 1 to 5, wherein Y contains at least one polyether radical, polyester radical, hydrocarbyl radical and/or polysiloxane radical.

7. Process for preparing a reaction product containing amido amine groups according to any of Claims 1 to 6, wherein
i) at least one diisocyanate R²(NCO)₂ is reacted with at least one monoalcohol Y-OH to form a urethane of the general formula (III)
Y-O-CO-NH-R²-NCO (III)
where
R² and Y are as defined in Claims 1 to 6 and
ii) p urethanes of the general formula (III) are reacted to give a reaction product containing amido amine groups, comprising one or more species of the general formula (I)
(R¹-X)ₚ-Z¹-(XH)_{y} (I)
where y, p, R¹, X and Z¹ are as defined in Claims 1 to 6, and
wherein step ii) is conducted in one stage ii a) or in a stage sequence ii-b),
in stage ii-a) p urethanes of the general formula (III) are reacted with a species of the general formula (IV) according to Claim 3 which is free of urethane groups and reactive toward isocyanate groups, and
in stage ii-b) p urethanes of the general formula (III) are reacted with an intermediate I containing at least one HX group reactive toward isocyanate groups with X as defined for the general formula (I) to form an intermediate J, wherein I is a Michael addition product of at least one component A with a component B or C, and then the intermediate J formed is reacted in an amidation reaction with a component B or C.

8. Process according to Claim 7, wherein the diisocyanate R²(NCO)₂ is used in step i) relative to the monoalcohol Y-OH in a molar ratio of at least 1.1:1.0 and the diisocyanate R²(NCO)₂ that has not been converted to the urethane of the general formula (III) in step i) is removed from the reaction mixture, preferably by distillation, prior to the performance of step ii).

9. Process according to either of Claims 7 and 8, wherein the diisocyanate R²(NCO)₂ corresponding to the R² radical has two isocyanate groups of different reactivity.

10. Process according to Claim 9, wherein the diisocyanate R²(NCO)₂ is selected from the group consisting of toluene 2,4-diisocyanate and isophorone diisocyanate.

11. Wetting agent and dispersant comprising a reaction product containing amido amine groups, according to any of Claims 1 to 6 or prepared by a process according to any of Claims 7 to 10.

12. Composition comprising a reaction product containing amido amine groups, according to any of Claims 1 to 6 or prepared by a process according to Claims 7 to 10.

13. Use of a reaction product containing amido amine groups according to any of Claims 1 to 6 or prepared by a process according to any of Claims 7 to 10 as an additive, preferably as a wetting agent or dispersant or dispersion stabilizer or viscosity reducer or compatibilizer in coatings, especially varnishes, plastics, pigment pastes, sealants, cosmetics, ceramics, adhesives, potting compounds, spackling compounds, printing inks and other inks.

14. Reaction product containing amido amine groups which is a species of the general formula (I) according to Claim 1.

15. Reaction product containing amido amine groups, comprising one or more salt(s) and/or one or more quaternization product(s) of the species of the general formula (I) according to Claim 1.

16. Reaction product containing amido amine groups, comprising one or more modification products of the species of the general formula (I) according to Claim 1, wherein the one or more tertiary amino groups of the general formula (I) have been converted to amine oxides with oxygen and/or peroxo compounds and/or XH groups still present in the general formula (I) have been reacted with carboxylic anhydrides.

## Revendications

1. Produit de réaction contenant des groupes amidoamine, contenant une ou plusieurs espèces selon la formule générale (I)
(R¹-Xₚ-Z¹-(XH)_{y} (I)
dans laquelle p + y = w, et
w représente un nombre entier de 1 à 10,
p représente un nombre entier de 1 à 10,
y représente un nombre entier de 0 à 9, et
les X représentent indépendamment les uns des autres O, NH et/ou NZ², et les XH représentent indépendamment les uns des autres un groupe hydroxyle OH, un groupe amino primaire NH₂ et/ou un groupe amino secondaire NHZ²,
les Z² représentant indépendamment les uns des autres un radical organique ramifié ou non ramifié, saturé ou insaturé G(U)ₐ, les U représentant indépendamment les uns des autres un groupe hydroxyle, un groupe amino primaire ou un groupe amino secondaire, et
a représentant un nombre entier de 0 à 9,
avec p + y + a ≤ 10, et
G représentant un radical organique ramifié ou non ramifié, saturé ou insaturé,
les p radicaux R¹ représentent indépendamment les uns des autres un radical de formule générale (II)
Y-O-CO-NH-R²-NH-CO (II)
dans laquelle les p radicaux Y représentent indépendamment les uns des autres un radical organique ramifié ou non ramifié, saturé ou insaturé, de 1 à 1 000 atomes de carbone, qui ne contient pas de groupe hydroxyle, pas de groupe amino primaire et pas de groupe amino secondaire,
les p radicaux R² représentent indépendamment les uns des autres un radical organique ramifié ou non ramifié, saturé ou insaturé, de 6 à 20 atomes de carbone,
Z¹ représente un radical organique ramifié ou non ramifié, saturé ou insaturé, contenant au moins deux atomes de carbone, qui comprend au moins un groupe amide et au moins un groupe amino tertiaire.

2. Produit de réaction contenant des groupes amidoamine selon la revendication 1, contenant au moins 40 % en poids, de préférence au moins 60 % en poids, de manière particulièrement préférée au moins 90 % en poids, d'une ou de plusieurs espèces de la formule générale (I).

3. Produit de réaction contenant des groupes amidoamine selon la revendication 1 ou 2, dans lequel les espèces réactives avec les groupes isocyanate, exemptes de groupes uréthane, correspondant à la formule (I), selon la formule générale (IV)
(HX)ₚ-Z¹-(HX)_{y} (IV)
avec p, y, X et Z¹ tels que définis pour la formule générale (I),
sont obtenues par mise en réaction d'un ou de plusieurs composants A avec un ou plusieurs composants B et/ou C, le composant A étant choisi dans le groupe constitué par les acides carboxyliques éthyléniquement insaturés, leurs esters et leurs halogénures d'acides, au moins une double liaison C=C et au moins une double liaison C=O étant présentes sous forme conjuguée, et la double liaison C=O étant choisie dans le groupe constitué par les acides carboxyliques, les esters d'acides carboxyliques et les halogénures d'acides carboxyliques ;
le composant B se présentant selon la formule générale (V)
(R³)ₓ-HN-(R⁴)_{z} (V)
avec x + z = 2, et
x représentant un nombre entier de 0 à 2,
z représentant un nombre entier de 0 à 2, et
les R³ représentant indépendamment les uns des autres H ou un radical organique ramifié ou non ramifié, saturé ou insaturé, de 1 à 12 atomes de carbone, et
les R⁴ représentant indépendamment les uns des autres un radical organique ramifié ou non ramifié, saturé ou insaturé, contenant 2 à 12 atomes de carbone et 1 à 3 groupes amino tertiaires ; et
le composant C se présentant selon la formule générale (VI)
(R⁵)ₖ-HN-(R⁶)ₙ (VI)
avec k + n = 2, et
k représentant un nombre entier de 0 à 1,
n représentant un nombre entier de 1 à 2, et
R⁵ représentant H ou un radical organique ramifié ou non ramifié, saturé ou insaturé, de 1 à 12 atomes de carbone, et
les R⁶ représentant indépendamment les uns des autres un radical organique ramifié ou non ramifié, saturé ou insaturé, contenant 2 à 12 atomes de carbone et 1 à 4 groupes hydroxyle.

4. Produit de réaction contenant des groupes amidoamine selon la revendication 3, dans lequel le composant A est un ester d'acide carboxylique éthyléniquement insaturé.

5. Produit de réaction contenant des groupes amidoamine selon l'une quelconque des revendications 1 à 4, dans lequel le diisocyanate R²(NCO)₂ correspondant au radical R² comprend deux groupes isocyanate de réactivité différente.

6. Produit de réaction contenant des groupes amidoamine selon l'une quelconque des revendications 1 à 5, dans lequel Y contient au moins un radical polyéther, un radical polyester, un radical hydrocarboné et/ou un radical polysiloxane.

7. Procédé de fabrication d'un produit de réaction contenant des groupes amidoamine selon l'une quelconque des revendications 1 à 6, selon lequel
i) au moins un diisocyanate R²(NCO)₂ est mis en réaction avec au moins un monoalcool Y-OH avec formation d'un uréthane de formule générale (III)
Y-O-CO-NH-R²-NCO (III)
dans laquelle
R² et Y sont tels que définis dans les revendications 1 à 6, et
ii) p uréthanes de formule générale (III) sont transformés en un produit de réaction contenant des groupes amidoamine, contenant une ou plusieurs espèces selon la formule générale (I)
(R¹-X)ₚ-Z¹-(XH)_{y} (I)
dans laquelle y, p, R¹, X et Z¹ sont tels que définis dans les revendications 1 à 6, et
l'étape ii) est réalisée en une étape ii-a) ou en une succession d'étapes ii-b),
lors de l'étape ii-a), p uréthanes de formule générale (III) étant mis en réaction avec une espèce réactive avec les groupes isocyanate, exempte de groupes uréthane, selon la formule générale (IV) selon la revendication 3, et
lors de l'étape ii-b), p uréthanes de formule générale (III) étant mis en réaction avec un intermédiaire I, qui contient au moins un groupe HX réactif avec les groupes isocyanate, X étant tel que défini pour la formule générale (I), avec formation d'un produit intermédiaire J, I étant un produit d'addition de Michael d'au moins un composant A avec un composant B ou C, puis le produit intermédiaire J formé étant mis en réaction dans une réaction d'amidation avec un composant B ou C.

8. Procédé selon la revendication 7, dans lequel, à l'étape i), le diisocyanate R²(NCO)₂ est utilisé par rapport au monoalcool Y-OH en un rapport molaire d'au moins 1,1:1,0, et le diisocyanate R²(NCO)₂ non transformé en l'uréthane de formule générale (III) à l'étape i) est éliminé du mélange réactionnel, de préférence par distillation, avant la réalisation de l'étape ii).

9. Procédé selon l'une quelconque des revendications 7 ou 8, dans lequel le diisocyanate R²(NCO)₂ correspondant au radical R² comprend deux groupes isocyanate de réactivité différente.

10. Procédé selon la revendication 9, dans lequel le diisocyanate R²(NCO)₂ est choisi dans le groupe constitué par le diisocyanate de 2,4-toluène et le diisocyanate d'isophorone.

11. Agent mouillant et dispersant contenant un produit de réaction contenant des groupes amidoamine selon l'une quelconque des revendications 1 à 6 ou fabriqué par un procédé selon les revendications 7 à 10.

12. Composition contenant un produit de réaction contenant des groupes amidoamine selon l'une quelconque des revendications 1 à 6 ou fabriqué par un procédé selon les revendications 7 à 10.

13. Utilisation d'un produit de réaction contenant des groupes amidoamine selon l'une quelconque des revendications 1 à 6 ou fabriqué par un procédé selon les revendications 7 à 10 en tant qu'additif, de préférence en tant qu'agent mouillant ou agent dispersant ou stabilisateur de dispersion ou réducteur de viscosité ou agent de compatibilisation dans des revêtements, notamment des vernis, des matières plastiques, des pâtes pigmentaires, des agents d'étanchéité, des cosmétiques, des céramiques, des adhésifs, des matériaux de scellement, des mastics, des encres d'impression et des encres.

14. Produit de réaction contenant des groupes amidoamine, dans lequel celui-ci est une espèce de formule générale (I) selon la revendication 1.

15. Produit de réaction contenant des groupes amidoamine, contenant un ou plusieurs sels et/ou un ou plusieurs produits de quaternisation des espèces de formule générale (I) selon la revendication 1.

16. Produit de réaction contenant des groupes amidoamine, contenant un ou plusieurs produits de modification des espèces de formule générale (I) selon la revendication 1, le ou les groupes amino tertiaires de la formule générale (I) ayant été transformés avec de l'oxygène et/ou des composés peroxo en aminoxydes, et/ou des groupes XH encore présents dans la formule générale (I) ayant été mise en réaction avec des anhydrides d'acides carboxyliques.
